# EUROPÄISCHE PATENTSCHRIFT

(11) **EP 2 443 503 B1**
(45) Veröffentlichungstag und Bekanntmachung des Hinweises auf die Patenterteilung: **23.12.2015**
(21) Anmeldenummer: 10734059.8
(22) Anmeldetag: 17.06.2010
(51) Int. Cl.: G02B 21/00, A61B 5/00, G01N 21/64

(54) **VORRICHTUNG UND VERFAHREN FÜR DIE MEHR-PHOTONEN-FLUORESZENZMIKROSKOPIE ZUR GEWINNUNG VON INFORMATIONEN AUS BIOLOGISCHEM GEWEBE**
DEVICE AND METHOD FOR MULTI-PHOTON FLUORESCENCE MICROSCOPY FOR OBTAINING INFORMATION FROM BIOLOGICAL TISSUE
DISPOSITIF ET PROCÉDÉ POUR LA MICROSCOPIE PAR FLUORESCENCE MULTIPHOTONIQUE VISANT À RECUEILLIR DES INFORMATIONS DE TISSUS BIOLOGIQUES

(30) Priorität: 17.06.2009 DE 102009029831
(43) Veröffentlichungstag der Anmeldung: 25.04.2012
(73) Patentinhaber: W.O.M. World of Medicine GmbH, 10587 Berlin (DE)
(72) Erfinder: SCHÖNBORN, Karl-Heinz, 12355 Berlin (DE)
(74) Vertreter: Maikowski & Ninnemann
(86) Internationale Anmeldenummer: PCT/EP2010/058576
(87) Internationale Veröffentlichungsnummer: WO 2010/146134

(56) Entgegenhaltungen:
- EP-A2- 1 929 939
- WO-A1-2007/054495
- DE-A1- 10 065 146
- US-A- 5 459 325
- US-B1- 6 249 630

## Beschreibung

Die Erfindung betrifft eine Vorrichtung für die Mehr-Photonen-Fluoreszenzmikroskopie zur Gewinnung von Informationen aus biologischem Gewebe nach dem Oberbegriff des Anspruchs 1 sowie ein Verfahren für die Mehr-Photonen-Fluoreszenzmikroskopie.

Eine derartige Vorrichtung weist eine Lasereinheit zur Erzeugung einer Anregungsstrahlung, eine Optikeinheit, die ausgebildet ist, die Anregungsstrahlung zur Erzeugung eines optischen Signals zu formen und an unterschiedlichen Orten in oder an einem zu untersuchenden Objekt zu fokussieren, ein Detektormodul zur Erfassung des optischen Signals aus dem Bereich des Objekts sowie ein Signalverarbeitungs- und Steuerungsmodul zur signaltechnischen und algorithmischen Verarbeitung der genannten optischen Signale, zu deren Umwandlung in ein diagnostisch auswertbares Bildsignal und zur Steuerung der gesamten Anlage auf.

Bei der Mehr-Photonen-Fluoreszenzmikroskopie (kurz: Mehr-Photonen-Mikroskopie) werden so genannte Mehr-Photonen-Mikroskope eingesetzt, bei denen es sich um spezielle Lichtmikroskope aus der Gruppe der Laser-Scanning-Mikroskope handelt. Hochaufgelöste mikroskopische Bilder werden dabei erzeugt durch Ausnutzung der so genannten Mehr-Photonen-Fluoreszenz (meist Zwei-Photonen-Fluoreszenz) oder der Erzeugung höherer Harmonischer, beispielsweise der Frequenzverdopplung oder - verdreifachung und daraus resultierend der Erzeugung der zweiten bzw. dritten Harmonischen (SHG: second harmonic generation; THG: third harmonic generation) des eingestrahlten Anregungslichtes.

Bei der Mehr-Photonen-Mikroskopie werden mit Hilfe einer starken, fokussierten Anregungsstrahlung, meist generiert durch einen Laser, nichtlineare optische Effekte in einem zu untersuchenden Gewebe erzeugt, die auf dem Zusammenspiel mehrerer gleichzeitig in einem Molekül eintreffender Photonen (Lichtteilchen) beruhen. Die Stärke des erzeugten Signals steigt dabei nicht linear mit der Zahl der pro Zeiteinheit eingestrahlten Photonen, sondern mit dem Quadrat (bei Zwei-Photonen-Effekten) oder der dritten Potenz (bei Drei-Photonen-Effekten). Die Arbeitsweise eines Mehr-Photonen-Mikroskops ähnelt bezüglich der Einstrahlung der Anregungsstrahlung in das Gewebe der eines konfokalen Laser-Scanning-Mikroskops. Beim konfokalen Mikroskop wird, anders als beim Multiphotonen-Mikroskop, remittierte Primärstrahlung und nicht Sekundärstrahlung zur Bilderzeugung verwendet. Ersteres Gerät weist ferner, anders als letzteres, im Signal-Detektionskanal ein "Pin-Hole" (enge Blende zur Elimination von remittierter Strahlung von außerhalb des Laserfokus) auf. Während wegen der vorgenannten Besonderheiten konfokale Laser-Scanning-Mikroskope eine Eindringtiefe je nach Präparat von 50-80 µm aufweisen, können mit der Mehr-Photonen-Mikroskopie tiefere Bereiche, z. B. bis zu 200 µm, in sehr günstigen Fällen sogar bis zu 1000 µm abgebildet werden, so dass aussagekräftige(re) Aufnahmen von lebendem Gewebe, beispielsweise von Hautschichten eines Menschen, möglich sind.

Das am weitesten verbreitete Verfahren für die Mehr-Photonen-Mikroskopie ist die Zwei-Photonen-Fluoreszenzmikroskopie (kurz: Zwei-Photonen-Mikroskopie). Während bei der herkömmlichen (Ein-Photonen-)Fluoreszenzmikroskopie in einem fluoreszierenden Molekül ein Elektron durch die Absorption jeweils eines Photons angeregt, also in einen höheren Energiezustand versetzt wird, wird bei der Zwei-Photonen-Fluoreszenzmikroskopie die Anregung des Elektrons durch die gleichzeitige oder nahezu gleichzeitige Absorption zweier Photonen hervorgerufen (Zwei-Photonen-Absorption).

Bei der Drei-Photonen-Mikroskopie erfolgt die Anregung entsprechend durch drei gleichzeitig oder nahezu gleichzeitig eintreffende Photonen.

Fluoreszenz entsteht, wenn Farbstoffe einfallende (anregende) Photonen absorbieren und in der Folge ein anderes Photon wieder abgeben. Durch die anregenden Photonen wird ein Elektron auf ein höheres Energieniveau gehoben und die Photonenenergie damit zwischengespeichert. Bei normaler Fluoreszenzmikroskopie geschieht diese Anregung durch genau ein Photon. Das Elektron bleibt für einige hundert Picosekunden bis zu mehreren Nanosekunden auf dem höheren Energieniveau, bevor es wieder zurück fällt und dabei ein neues, längerwelliges, energieärmeres Photon aussendet. Wenn etwa mit blauem Licht angeregt wird, entsteht beispielsweise grüne Fluoreszenz, beispielsweise bei Fluorescein. Bei der Zwei-Photonen-Mikroskopie erfolgt die Anregung eines Elektrons genau durch zwei Photonen, die in der Summe die gleiche Energie aufweisen wie das eine Anregungsphoton der normalen Fluoreszenzmikroskopie. Voraussetzung für die Anregung ist jedoch, dass die zwei Photonen gleichzeitig - innerhalb einer Attosekunde (10⁻¹⁸ s) - eintreffen, da kein stabiles Zwischenenergieniveau des anzuregenden Elektrons existiert.

Bei normaler Fluoreszenzmikroskopie hat das anregende Photon eine kürzere Wellenlänge und damit mehr Energie als das abgestrahlte Photon. Im Gegensatz hierzu wird bei der Mehr-Photonen-Anregung mit Photonen angeregt, die eine deutlich größerer Wellenlänge und somit pro Photon weniger Energie aufweisen als die ausgesandten Photonen. Beispielsweise kann auf diese Weise dunkelrotes oder infrarotes Licht zur Anregung eingesetzt werden, um grüne Fluoreszenz zu erzeugen. Dies ist möglich, weil zwei oder mehr anregende Photonen zur Erzeugung nur eines ausgesandten Photons führen. Bei der Zwei-Photonen-Anregung beträgt die Anregungswellenlänge in etwa das Doppelte der normalerweise verwendeten Anregungswellenlänge, bei Drei-Photonen-Anregung das Dreifache usw.

Das Grundkonzept der Zwei-Photonen-Fluoreszenzmikroskopie ist in der Veröffentlichung W. Denk, J.H. Strickler, W.W. Webb "Two-Photon Laser Scanning Fluorescence Microscopy", Science, Vol. 248, S. 73-76 (6. April 1990) beschrieben.

Aus der US 5,034,613 ist eine Vorrichtung für die Mehr-Photonen-Fluoreszenzmikroskopie bekannt, bei der eine durch einen Laser erzeugte Anregungsstrahlung durch im Strahlengang befindliche bewegliche Spiegel auf ein zu untersuchendes Objekt gerichtet wird. Um dabei eine Anregung an unterschiedlichen Orten des Objekts zu erreichen und auf diese Weise ein pixelweise angeregtes Bild zu erzeugen, wird der Anregungsstrahl durch Verkippung der beweglichen Spiegel in seiner Lage so verändert, dass der Fokuspunkt der Anregungsstrahlung sich durch das Objekt bewegt, dieses Ort für Ort anregt und dadurch Ort für Ort Signale in dem Objekt erzeugt. Die dadurch entstehende Sekundärstrahlung (bestehend aus einer Fluoreszenzstrahlung und etwaig erzeugter höherer Harmonischer der Anregungsstrahlung) wird aufgefangen und detektiert, um anhand der Signale aus den einzelnen Orten ein vollständiges Bild in einer oder in mehreren Ebenen des Objekts zu erzeugen.

Mit der aus der US 5,034,613 bekannten Vorrichtung können im Wesentlichen nur Schnittbilder aus einem kleinen Ausschnitt des zu untersuchenden Objektes erzeugt werden. Dies ist dadurch bedingt, dass bei der Zwei-Photonen-Mikroskopie wegen der notwendigen hohen Intensitäten zur Anregung, die eine Fokussierung der Anregungsstrahlung auf einen Fokusdurchmesser von 0,5 µm bis maximal ca. 3 µm erforderlich machen, große Aperturen der verwendeten Optik nötig sind (dies ergibt sich aus den Gesetzen der Strahlprodukt-Erhaltung bei optischer Abbildung von Laserstrahlen). Diese Aperturen weisen eine numerische Apertur von NA=0,4 bis NA=1 (bzw. bei Verwendung eines Immersionsfluids bis NA=1,45) auf, was einem Kegelvollwinkel des fokussierten Strahls von ca. 50° bis 135° entspricht. Solche großaperturigen Strahlbündel können nur durch hochvergrößernde Mikroskopobjektive oder vergleichbare komplexe Optiken fokussiert werden, die unvermeidlich nur vergleichsweise geringe Gesichtsfelder mit einem Durchmesser von ca. 0,5 mm bis 1 mm aufweisen (unter einem Gesichtsfeld ist hier das maximale Feld zu verstehen, dass ein abgelenkter Anregungsstrahl überstreichen kann). Das heißt mit anderen Worten: Bei den für die Zwei-Photonen-Mikroskopie notwendigen großen Aperturen zur Fokussierung der Anregungsstrahlung auf das Objekt ist die Fläche, die die Anregungsstrahlung durch Strahlablenkung überstreichen kann, zwangsläufig beschränkt. Durch Strahlablenkung unter Verwendung von Dreh- und Kippspiegeln können nur Felder mit einem Durchmesser von maximal 1 mm angeregt werden, so dass die aufnehmbaren Bilder auf Kantenlängen von maximal 1 mm beschränkt sind.

Um die Zwei-Photonen-Mikroskopie beispielsweise für die Untersuchung einer Hautschicht auf pathologische Veränderungen einzusetzen, sind solche Bilder häufig zu klein.

Herkömmlich wird bei der Zwei-Photonen-Mikroskopie zunächst ein lateraler Scan einer Ebene (Hautschicht) durchgeführt und anschließend die Fokustiefe für die Aufnahme weiterer, z.B. tieferliegender Hautschichten neu eingestellt. Auf diese Weise wird sukzessive eine Abfolge übereinander liegender Schichten durch die Haut aufgenommen. Wünschenswert wären dagegen aus Anwendersicht im Falle des medizinischen Einsatzes vertikale Schnittbilder durch die Haut, die der in der Histopathologie üblichen Schnittlage entsprechen, die die ärztlichen Untersucher gewohnt sind und die ihrem diagnostischen Blick entsprechen.

Insgesamt wären demnach Bilder aus der Haut in vertikaler Schnittlage wünschenswert, die beispielsweise eine sich über eine Länge von mehreren mm oder gar cm erstreckende Läsion vollständig abbilden können.

Aus der EP 1 929 939 A2 ist eine endoskopisch einsetzbare Vorrichtung für die Mehr-Photonen-Mikroskopie bekannt, bei der die Spitze einer als Lichtleiter dienenden optischen Faser mit einer daran angeordneten miniaturisierten Fokussieroptik in einem Endoskop beweglich ist, um eine durch einen Laser erzeugte Anregungsstrahlung auf ein Objekt zu lenken. Dadurch, dass an der Spitze des Lichtleiters lediglich eine Optik kleiner Abmessung und demzufolge auch kleiner Apertur eingesetzt werden kann, ist die erreichbare örtliche Auflösung begrenzt, da nur vergleichsweise große Fokusdurchmesser zur örtlichen Anregung erzielbar sind. Zudem verwendet die Anordnung der EP 1 929 939 A2 für die Hinleitung der Anregungsstrahlung und die Rückleitung der aus dem Objekt aufgenommenen optischen Signale denselben Lichtleiter, was zum einen hohe Anforderungen an den Lichtleiter stellt (Transmission von ultrakurzgepulster Laserstrahlung hoher Strahlqualität, d.h. im TEM00-Mode) und zum anderen nachteilig für die Übertragungsqualität und -ausbeute der empfangenen Signale ist. Die transmittierte Anregungsstrahlung wird zusätzlich durch die Faserdispersion in ihrer Qualität verschlechtert, z.B. durch Verlängerung der Pulsdauer oder das so genannte "Chirping". Darüber hinaus muss bei der Bewegung der Fokussieroptik zur Aufnahme eines vollständigen Bildes immer der Lichtleiter mitbewegt werden, was die räumliche Bewegung aufwendig macht und gleichzeitig beschränkt, da Schnittbilder von großer lateraler Ausdehnung nicht aufgenommen werden können.

Bei der EP 1 929 939 A2 wird eine Anregungsstrahlung durch einen Femtosekundenlaser erzeugt und über einen Shutter, einen Abschwächer und einen dichroitischen Teilerspiegel einer Faser zugeführt. Bei dieser wird die Anregungsstrahlung mit der eigentlichen Anregungsfrequenz über die optische Faser unmittelbar dem Objekt zugeführt.

Die WO 2007/054495 A1 bezieht sich auf einen mechanischen Aufbau zum Scannen eines Objekts, bei der eine Übertragung einer von einer Lasereinheit erzeugten Anregungsstrahlung mittels einer Faser nicht vorgesehen ist. Ein ähnlicher Aufbau ist auch aus der US 5,459,325 bekannt.

Bei einer aus der DE 100 65146 A1 bekannten Vorrichtung wird eine Anregungsstrahlung durch einen Femtosekundenlaser bei einer Wellenlänge von 800 nm erzeugt und über einen optischen Gelenkarm mit reflektierenden Elementen einer Scanneroptik zugeführt.

Die US 6,249,630 beschreibt eine Übertragung zwischen einer optischen Quelle und einem optischen Gerät, bei der vor Einkoppeln in eine optische Faser eine Dehnung von optischen Pulsen in einem so genannten Stretcher vorgesehen ist, um verbreiterte optische Pulse mit einer reduzierten Spitzenleistung ("chirped optical pulses") zu erhalten. Diese Pulse sind am Ausgang der Faser wiederum zu komprimieren, um die Verbreiterung rückgängig zu machen und die Pulse in ihre Ausgangsform zurückzuführen. Hierzu ist ein so genannter Kompressor vorgesehen.

Der vorliegenden Erfindung liegt die Aufgabe zugrunde, eine Vorrichtung und ein Verfahren für die Mehr-Photonen-Fluoreszenzmikroskopie zur Gewinnung von Informationen aus biologischem Gewebe zu schaffen, die bei einem großen Gesichtsfeld die Aufnahme von insbesondere vertikalen Schnittbildern in einem Objekt ermöglichen und dabei einfach aufgebaut und zuverlässig in ihrem Betrieb sind. Dabei sollten die zeitlichen, spektralen und polarisationsbezogenen Strahleigenschaften der Anregungsstrahlung auf ihrem Strahlengang nicht oder nur minimal verschlechtert werden, und die Handhabung (Ergonomie) und die Einsatzfähigkeit sollten verbessert werden.

Diese Aufgabe wird durch einen Gegenstand mit den Merkmalen des Anspruchs 1 gelöst.

Erfindungsgemäß ist dabei vorgesehen, dass die Optikeinheit ausgebildet und vorgesehen ist, zur Erzeugung des optischen Signals an unterschiedlichen Orten in oder an dem Objekt zumindest in einer Richtung relativ zum Objekt bewegt zu werden.

Die Erfindung geht von dem Grundgedanken aus, eine so genannte fliegende Optik zu verwenden. Die Optikeinheit zur Fokussierung der Anregungsstrahlung und zur Anregung einer Sekundärstrahlung an einem Ort in oder an dem Objekt ist nicht fest angeordnet, sondern wird insgesamt bewegt, um unterschiedliche Orte eines Objekts zeitlich nacheinander anzuregen. Die örtliche Anregung erfolgt somit nicht durch Strahlablenkung unter Verwendung von Dreh- und Kippspiegeln, sondern durch eine ein- oder mehrdimensionale Bewegung der Optikeinheit im Ganzen. Vorteilhafterweise sollte sich bei der Bewegung der Optikeinheit zur Erzeugung des optischen Signals die optische Achse der auf das Objekt fallenden Anregungsstrahlung dabei nicht ändern, so dass - im Gegensatz zu einer Verwendung von Dreh- und Kippspiegeln - die Anregungsstrahlung immer unter demselben Winkel auf das Objekt trifft.

Dadurch, dass auf eine Strahlablenkung mittels Dreh- und Kippspiegeln verzichtet wird, können große Gesichtsfelder erreicht werden. Das bedeutet, dass theoretisch Felder beliebiger Größe angeregt werden können, um auf diese Weise Bilder mit großen Kantenlängen aus dem zu untersuchenden Objekt zu erzeugen. Dies ermöglicht beispielsweise, Schnittbilder der menschlichen Haut aufzunehmen, die Läsionen vollständig abbilden können.

Erfindungsgemäß ist die Optikeinheit in horizontaler Richtung und/oder in vertikaler Richtung relativ zu einer der Optikeinheit zugewandten Oberfläche des Objekts bewegbar. Zur Aufnahme eines Schnittbildes wird die Optikeinheit zum einen entlang der Oberfläche des Objektes, beispielsweise entlang der Hautoberfläche eines Patienten verfahren, wobei die Bewegung auch zweidimensional in X- und Y-Richtung entlang der Hautoberfläche und gleichzeitig in Z-Richtung vertikal zur Hautoberfläche zur Signalerzeugung in einem dreidimensionalen Raum erfolgen kann. Beim Verfahren werden nacheinander unterschiedliche Orte des Objekts angeregt, und die im Objekt erzeugte Sekundärstrahlung - bestehend aus im Objekt erzeugter Fluoreszenzstrahlung und durch nichtlineare Effekte erzeugte Harmonische der Anregungsstrahlung (SHG: second harmonic generation = Erzeugung der ersten Oberwelle) - wird als optisches Signal aufgenommen.

Um auf einfache Weise eine Bewegung des Fokus in vertikaler Richtung zu erreichen, kann vorgesehen sein, nicht die gesamte Optikeinheit (umfassend beispielsweise ein dichroitisches Element zur Trennung von Anregungsstrahlung und empfangenen optischen Signalen), sondern lediglich ein Objektiv beweglich auszugestalten. Das Objektiv, das beispielsweise eine optische Linse zur Fokussierung aufweisen kann, ist damit zur übrigen Optikeinheit zumindest in vertikaler Richtung beweglich, um den Fokus innerhalb des anzuregenden Objektes in vertikaler Richtung zu bewegen und Signale in Form einer Sekundärstrahlung an unterschiedlichen, vertikal versetzten Orten zu erzeugen.

Zur pixelweisen Erzeugung eines vertikalen Schnittbildes kann die Optikeinheit zumindest abschnittsweise kontinuierlich in horizontaler Richtung und/oder in vertikaler Richtung relativ zu dem Objekt verfahrbar sein. Die Optikeinheit wird so entlang vordefinierter Aufnahmelinien (Scanlinien) bewegt und scannt das Objekt entlang dieser Aufnahmelinien, wobei nacheinander, beispielsweise durch Triggerung der Anregungsstrahlung zur zeitabhängigen Belichtung, Orte entlang der Aufnahmelinie innerhalb des Objektes zur Emission von Sekundärstrahlung angeregt und Signale aus diesen Orten empfangen werden. Die Signale von einem Ort ergeben dann einen Pixel des aufzunehmenden Bildes, wobei mittels der Aufnahmelinien (Scanlinien) das Objekt entlang einer zu betrachtenden Ebene so gerastert wird, dass sich ein vollständiges, beispielsweise zur medizinischen Diagnose auswertbares Bild ergibt.

Die Vorrichtung wird zur Mehr-Photonen-Mikroskopie eingesetzt und ist dabei modular aufgebaut. Als zentrale Einheit weist die Vorrichtung eine Steuer- und Verarbeitungseinheit auf, die über einen Tragarm mit einem so genannten Patientenmodul verbunden ist. Unter "Tragarm" sei hier und im Folgenden eine mechanische Haltevorrichtung verstanden, die eine leichtgängige Beweglichkeit des Patientenmoduls, ggf. mit Gewichtsausgleich, während der Positionierung der Messeinrichtung im Verhältnis zum Patienten oder zur Probe ermöglicht sowie eine Fixierung für den Zeitraum der Fluoreszenzaufnahme. Die Steuer- und Verarbeitungseinheit ist stationär ausgebildet, dient der zentralen Steuerung der Vorrichtung und der Verarbeitung der empfangenen Signale und weist auch die Lasereinheit zur Erzeugung der Anregungsstrahlung auf. Das Patientenmodul ist über den Tragarm relativ zur Steuer- und Verarbeitungseinheit beweglich und kann an einem zu untersuchenden Objekt so platziert werden, dass die Anregungsstrahlung in geeigneter Weise auf das Objekt, beispielsweise die Haut eines Patienten, fällt und erzeugte Signale aufgenommen werden können. Die Optikeinheit ist Teil des Patientenmoduls und innerhalb dessen beweglich, wobei das Patientenmodul einen für die Anregungsstrahlung und das optische Signal durchlässigen Kontaktabschnitt (beispielsweise eine an einem Gehäuse des Patientenmoduls angeordnete Glasscheibe) aufweist, der zur Untersuchung des Objekts mit dem Objekt in Kontakt zu bringen ist. Während der Kontaktabschnitt bei einer Aufnahme ortsfest an dem zu untersuchenden Objekt (beispielsweise unter Verwendung eines Immersionsfluids) anliegt, ist die Optikeinheit in horizontaler Richtung und/oder in vertikaler Richtung relativ zu dem Kontaktabschnitt und damit auch zu dem Objekt bewegbar.

Die die Lasereinheit ist Teil der Steuer- und Verarbeitungseinheit und somit räumlich getrennt von dem Patientenmodul. Die von der Lasereinheit erzeugte Anregungsstrahlung wird über eine optische Faser hin zum Patientenmodul und zur Optikeinheit zur Einstrahlung auf das Objekt übertragen. Die optische Faser kann dabei entlang des Tragarms oder auch innerhalb des Tragarms zum Patientenmodul verlegt sein.

Um für die Zwei- oder Mehr-Photonen-Mikroskopie ein gleichzeitiges Eintreffen zweier oder mehr Photonen im Fokuspunkt zu erreichen und auf diese Weise die Moleküle innerhalb des Objekts anzuregen, sind sehr hohe Photonendichten in der Anregungsstrahlung erforderlich. Diese können beispielsweise erzielt werden, indem ein gepulster Laser (Ultrakurzpulslaser) zur Erzeugung von Laserpulsen im Femtosekunden-Bereich insbesondere mit Modenkopplung eingesetzt wird. Solche Laser senden sehr kurze, intensive Laserpulse (mit Pulslängen im Femtosekundenbereich, z.B. 80-140 fs) aus, die z.B. 80-120 Millionen mal pro Sekunde wiederholt werden, so dass sich Pausen zwischen den Pulsen mit einer Länge von 8 bis 12,5 ns (= 8000000-12500000 fs) ergeben und die gesamte im Laser erzeugte Energie auf diese Weise in gepulster Form mit hoher Intensität in einem Bruchteil der Zeit abgegeben wird.

Die Lasereinheit erzeugt eine Anregungsstrahlung einer ersten Wellenlänge, z.B. 1560 nm. In dem Patientenmodul ist dann ein der Optikeinheit vorgeschalteter Frequenzverdoppler (z.B. in Form eines Frequenzverdopplungskristalls) angeordnet, der die Wellenlänge der Anregungsstrahlung halbiert (beispielsweise von 1560 nm auf 780 nm) und damit die Frequenz der Anregungsstrahlung verdoppelt. Dies hat den Vorteil, dass die Anregungsstrahlung mit einer vergleichsweise großen Wellenlänge von z.B. 1560 nm über eine geeignete optische Faser von der Lasereinheit hin zum Patientenmodul übertragen werden kann, wobei für einen solchen Wellenlängenbereich Fasern verfügbar sind, die eine Übertragung - auch unter Beibehaltung der Polarisation ("polarisationserhaltende Einmodenfasern") - ohne nennenswerte Beeinträchtigung der Strahlqualität ermöglichen. Der Frequenzverdoppler erzeugt dann die erste Oberwelle der übertragenen Anregungsstrahlung (z.B. 780 nm), die zur Anregung des Objekts verwendet wird.

Die Lasereinheit kann auch als so genannter Femtosekunden-Faserlaser mit herausgeführter Laserfaser ausgeführt sein, die sich bis zum Patientenmodul hin erstreckt. Auf diese Weise kann auf ein so genanntes "Pre Chirp" (eine Vorverzerrung der Anregungsstrahlung zur Kompensation der Dispersionseffekte, vor allem der Gruppengeschwindigkeitsdispersion, im optischen Weg von der Laserstrahlquelle bis zum Gewebe) verzichtet werden, weil das Ende der Laserfaser den Austrittsort der Anregungsstrahlung aus dem Laserresonator und damit die primäre Laserstrahlungsquelle darstellt. Im Patientenmodul erfolgt dann die Frequenzverdopplung der Anregungsstrahlung bzw. die Halbierung der Wellenlänge von 1560 nm auf 780 nm.

Die Idee, eine Lasereinheit zu verwenden, die eine Strahlung einer ersten Wellenlänge erzeugt, die dann nachträglich in eine Anregungsstrahlung mit einer anderen Wellenlänge gewandelt wird und bei der die primäre Strahlungsquelle, (sie erzeugt die erste Wellenlänge) in einer Geräteeinheit montiert ist und ihre Strahlung über eine optische Faser zu einer zweiten separaten Geräteeinheit überträgt, wo die Strahlung durch Wandlung in die Anregungswellenlänge überführt und dann weiterverwendet wird, stellt in diesem Zusammenhang auch ein eigenständiges Konzept dar, das bei verschiedensten Vorrichtungen für die Mehr-Photonen-Fluoreszenzmikroskopie zur Gewinnung von Informationen aus biologischem Gewebe eingesetzt werden kann. Eine solche Vorrichtung kann beispielsweise allgemein folgende Merkmale aufweisen:
- eine Lasereinheit zur Erzeugung einer Anregungsstrahlung,
- eine Optikeinheit, die ausgebildet ist, die Anregungsstrahlung zur Erzeugung eines optischen Signals an unterschiedlichen Orten in oder an einem zu untersuchenden Objekt zu fokussieren, und
- ein Detektormodul zur Erfassung des optischen Signals aus dem Bereich des Objekts,
wobei die Lasereinheit eine Strahlung einer ersten Wellenlänge erzeugt und über eine optische Faser zu der Optikeinheit überträgt, wo sie dann anschließend in die Anregungsstrahlung mit einer von der ersten Wellenlänge unterschiedlichen, zweiten Wellenlänge gewandelt wird.

Auf diese Weise kann die Lasereinheit beispielsweise eine Strahlung mit einer Wellenlänge von 1560 nm erzeugen, die dann in eine Anregungsstrahlung mit einer halbierten Wellenlänge von 780 nm gewandelt und der Optikeinheit zur Anregung des Objekts zugeführt wird.

Auch das Konzept des Linearscans durch Bewegen der Optikeinheit entlang einer vorbestimmten Aufnahmelinie (Scanlinie) zur Erzeugung eines zweidimensionalen Schnittbildes oder eines dreidimensionalen Volumenbildes ist unabhängig von der vorgenannten zweistufigen Strahlerzeugung ein eigenständiges erfinderisches Konzept.

Der Laserstrahl der Anregungsstrahlung kann beispielsweise ursprünglich linear polarisiert sein entsprechend einer transversalen Grundmode (TEM00), wobei zum Ausgleich von Inhomogenitäten der Strahl vor Erreichen der Optikeinheit beispielsweise durch Einfügen eines geeigneten Lambda-Viertel-Plättchens zirkular polarisiert werden kann.

Vorteilhafterweise ist die Optikeinheit einerseits zur Fokussierung der Anregungsstrahlung auf das Objekt und andererseits zur Kollektion des Zwei-Photonen-angeregten optischen Signals ausgebildet. Die Optikeinheit kann dabei mit einer optischen Faser verbunden sein, über die das aufgenommene optische Signal an das Detektormodul zur weiteren Verarbeitung übertragen wird. Für diese optische Faser (auch bezeichnet als "Kollektionsfaser") wird bevorzugt eine Multimodenfaser oder ein Faserbündel aus einer Vielzahl von Einzelfasem verwendet, die an den Enden gemeinsam gefasst sind und so jeweils eine kompakte Eintritts- bzw. Austrittsfläche der Fluoreszenzstrahlung bilden.

In dem Detektormodul, das vorteilhafterweise in die Steuer- und Verarbeitungseinheit integriert ist, aber auch unmittelbar in das Patientenmodul eingefügt sein kann, erfolgt die Signalverarbeitung und Bildbearbeitung. Um dabei sowohl Intensitätsinformationen als auch spektroskopische Informationen gewinnen zu können, kann die Signalverarbeitung mehrkanalig erfolgen, wobei das empfangene Signal in dem Detektormodul in mehrere unterschiedliche Signalanteile mit unterschiedlichen Wellenlängenbereichen aufgeteilt wird, die dann getrennt voneinander verarbeitet werden können. Auf diese Weise wird das Signal in unterschiedliche Signalbänder mit unterschiedlichen Wellenlängen aufgeteilt, wobei die Bänder abhängig von den gesuchten Informationen in geeigneter Weise gewählt werden können. Soll beispielsweise bestimmt werden, wo ein Spektrum in etwa ein Maximum aufweist, so kann das Verhältnis der spektralen Anteile in einem oberen Band (mit längeren Wellenlängen) und in einem unteren Band (mit kürzeren Wellenlängen) gebildet werden. Sollen bestimmte fluoreszierende Stoffe nachgewiesen werden, so kann ein Band gezielt den Wellenlängenbereich umfassen, in dem der gesuchte Stoff eine Fluoreszenzstrahlung emittiert. Beispielsweise können auf diese Weise zum Zwecke der photodynamischen Diagnostik (kurz PDD) oder photodynamischen Therapie (kurz PDT) Porphyrine (insbesondere das Protoporphyrin IX, PP IX) in Zellen nachgewiesen werden, die bei Anregung Fluoreszenzstrahlung in bestimmten Wellenlängenbereichen emittieren (PP IX beispielsweise u.a. bei ca. 630 nm). Aus dem Vorhandensein der Porphyrine kann dann auf den Gewebezustand, insbesondere auf krankhafte Gewebeneubildungen wie z.B. bei Krebs, geschlossen werden, wobei diese Information wiederum zur Steuerung im Rahmen der photodynamischen Therapie zur gezielten Zellschädigung oder -zerstörung verwendet werden kann.

Die Vorrichtung kann zur Abbildung sowohl körpereigener als auch körperfremder fluoreszierender Stoffe (so genannter Fluophore) eingesetzt werden. Natürliche in der menschlichen Haut vorkommende Fluophore sind dabei beispielsweise NAD(P)H, Collagen, Elastin, Trytophan, Flavine, Lipo-Pigmente, Keratin, HPD (Hämatoporphyrin und Derivate) sowie PP IX, die in unterschiedlichen Wellenlängenbereichen fluoreszieren und damit durch geeignete Auswahl der jeweils betrachteten Bänder erfasst werden können.

Um das empfangene Signal in Signalanteile zu zerlegen, weist das Detektormodul einen oder mehrere dichroitische Filterelemente auf, die die einfallende Strahlung wellenlängenabhängig reflektieren oder transmittieren und damit wellenlängenabhängig zerlegen.

Um auf einfache Weise unterschiedliche spektrale Bänder analysieren zu können, kann dabei vorgesehen sein, das eine oder die mehreren dichroitischen Filterelemente nach Art eines Baukastensystems austauschbar zu gestalten. Soll das empfangene Signal in bestimmten Bändern betrachtet werden, so wird der dafür geeignete Satz von dichroitischen Filterelementen, beispielsweise dichroitischen Spiegeln oder Prismen, gewählt und in das Detektormodul eingesetzt. Sollen andere Bänder betrachtet werden, kann ein anderer Satz von Filterelementen verwendet und die Messung entsprechend wiederholt werden. Dieser Filterwechsel kann manuell oder in der Art eines motorisch bewegten Filterrevolvers oder Filterwechselmagazins ausgeführt werden.

Denkbar ist in diesem Zusammenhang auch, mit der Vorrichtung hochaufgelöste Spektroskopie durchzuführen, also pixelweise ein vollständiges Spektrum aufzunehmen. Voraussetzung dafür ist, dass aus jedem Ort eine ausreichend große Anzahl an Photonen für ein ausreichend starkes, auswertbares Signal empfangen wird. Gegebenenfalls können hierfür Signale aus mehreren angeregten Orten integriert werden.

Zur Detektion der unterschiedlichen Signalanteile weist das Detektormodul beispielsweise - abhängig von der Anzahl der betrachteten Bänder - einen oder mehrere Detektoren auf, die beispielsweise als so genannte Sekundärelektronenvervielfacher (Photo Multiplier Tube, PMT), als CCD-Zeile, als CCD-Feld oder als SiPMT ("Silicon Photo Multiplier", d.h. Bauelemente mit Detektor-Feldern aus Photosignal-additiv verschalteten Avalanche-Photodioden) ausgebildet sind und der Umwandlung des empfangenen optischen Signals bzw. seiner einzelnen Signalanteile in elektronische Datensignale dienen.

Zur Bildverarbeitung kann das Detektormodul aus den unterschiedlichen Signalanteilen einerseits eine Helligkeitsinformation und andererseits eine spektroskopische Information generieren und als Schnittbild durch das zu untersuchende Objekt mit einer spektroskopischen Zusatzinformation ausgeben und beispielsweise auf einem Monitor anzeigen. Dabei kann beispielsweise aus der Gesamtheit der Signalanteile eine Helligkeitsinformation abgeleitet werden, die anhand des Bildkontrasts Strukturinformationen liefert. Dem überlagert werden können durch einen Nutzer wählbar und einstellbar spektrale Zusatzinformationen, die aus den einzelnen Signalanteilen und damit unterschiedlichen Wellenlängenbändern erhalten worden sind. Beispielsweise kann dem Helligkeitsbild so die Information überlagert und in Falschfarben dargestellt werden, an welchen Orten ein bestimmter fluoreszierender Stoff vorhanden ist oder an welchen Orten Signaloberwellen (z.B. SHG) durch strukturelle Gewebeeigenschaften (beispielsweise durch das Vorhandensein von Collagen in bestimmten Hautschichten) auftreten.

Die Aufgabe wird darüber hinaus durch ein Verfahren für die Mehr-Photonen-Fluoreszenzmikroskopie zur Gewinnung von Informationen aus biologischem Gewebe nach Anspruch 9 gelöst bei dem eine Lasereinheit eine Anregungsstrahlung erzeugt, eine Optikeinheit die Anregungsstrahlung zur Erzeugung eines optischen Signals an unterschiedlichen Orten in oder an einem zu untersuchenden Objekt fokussiert und ein Detektormodul das optische Signal aus dem Bereich des Objekts erfasst. Im Rahmen des Verfahrens ist vorgesehen, dass die Optikeinheit zur Erzeugung des optischen Signals in oder an dem Objekt zumindest in einer Richtung relativ zum Objekt bewegt wird.

Die vorangehend für die Vorrichtung beschriebenen Vorteile und Ausgestaltungen sind auf das Verfahren analog übertragbar.

Aus den empfangenen optischen Signalen kann an unterschiedlichen Orten des Objekts pixelweise ein Schnittbild des Objekts erzeugt werden, wobei zur pixelweisen Anregung das Objekt in getriggerter Weise mit der Anregungsstrahlung belichtet wird. Mit anderen Worten wird die Optikeinheit relativ zum Objekt entlang einer geeigneten, vorab festgelegten Aufnahmelinie (Scanlinie) verfahren, und dabei wird das Objekt sukzessive an einzelnen Orten, die jeweils gerade dem Ort des momentanen Fokus der Optikeinheit entsprechen, angeregt und somit belichtet, wobei die Triggerung ortsabhängig gesteuert und die Belichtungszeit, also die Zeit der Bestrahlung eines bestimmten Ortes, in geeigneter Weise eingestellt werden kann.

Die Triggerung der Anregungsstrahlung dient unter anderem für die Festlegung der Pixelgröße. Die Pixelgröße des aus den empfangenen, ortsabhängigen Signalen erhaltenen Bildes ist in horizontaler Richtung durch die Fokusbreite der Anregungsstrahlung und durch die Einstellung der Triggerung bestimmt, während in vertikaler Richtung die Pixelgröße durch die Taillenlänge der fokussierten Anregungsstrahlung gegeben ist. Durch eine Strahlaufweitung der Anregungsstrahlung und Einstellung der Triggerung ist die Pixelgröße dann einstellbar, wobei beispielsweise zur Spektroskopie vergleichsweise große Pixel verwendet werden können, um ein vergleichsweise intensitätsstarkes Signal zu erhalten, während zur hochaufgelösten Mikroskopie bevorzugt kleine Pixel eingesetzt werden. Dabei kann die Bilderzeugung (Mikroskopie) und die Spektroskopie zeitlich parallel während desselben Scanvorgangs ablaufen.

Denkbar ist in diesem Zusammenhang, die Pixelgröße in Stufen oder stufenlos zu verstellen, indem einerseits die Strahlaufweitung mittels einer Vergrößerungsumschaltung oder eines ZOOMs in einem Aufweitungsteleskop umgeschaltet und dadurch die Taillenlänge und damit die vertikale Ausdehnung des Pixels verstellt wird und andererseits durch Umschaltung der Triggerung die laterale Ausdehnung des Fokus entsprechend variiert wird.

Bei der Zwei-Photonen-Mikroskopie wird die Anregungsstrahlung wie oben beschrieben in Form eines Laserstrahls mit einer hohen Apertur in das Objekt (in der Regel ein Gewebe) hineinfokussiert, um einen kleinen Fokusdurchmesser und eine geringe Tiefenschärfe und damit ein kleines Fluoreszenzanregungsvolumen, d.h. eine hohe laterale und axiale Ortsauflösung zu erzielen. In Abkehr hiervon kann alternativ eine so genannten "homogenisierte Fluoreszenzanregung" verwendet werden, bei der es das Ziel ist, einen lateral begrenzten, allerdings axial (vertikal) ausgedehnten Bereich des Objekts so anzuregen, dass in axialer Richtung Objektschichten weitestgehend gleichgewichtig zumindest über einen bestimmten Tiefenbereich zum optischen Signal beitragen. Dieses optische Signal wird dann aufgenommenen und integriert und kann als einzelner Messwert, als Spektrum oder mehrkanalig mit separaten Spektralbanden ("Bandenspektroskopie") ausgewertet und weiterverarbeitet werden.

Zur Aufnahme wird die Optikeinheit ausschließlich in horizontaler (lateraler) Richtung (in X- oder in X- und Y-Richtung) zur Oberfläche des Objekts bewegt, und das optische Signal wird in vertikaler Richtung integriert. Es entsteht ein Messwertefeld, das kein Objektbild (Gewebebild) wie bei der herkömmlichen Zwei-Photonen-Mikroskopie darstellt, sondern eine lateral ortsaufgelöste Information über den Objektzustand insgesamt liefert.

Die homogenisierte Fluoreszenzanregung kann dadurch erreicht werden, dass die Anregungsstrahlung mit einer vergleichsweise kleinen Apertur in das Gewebe eingestrahlt und auf eine bestimmte Tiefe fokussiert wird. Der durch ein Objektiv der Optikeinheit, beispielsweise eine asphärische Linse, fokussierte Anregungsstrahl wird hierbei durch die Apertur, die Fokustiefe und den Fokusdurchmesser definiert. Die Fokustiefe wird zur homogenisierten Fluoreszenzanregung vorteilhafterweise auf einen Wert zwischen 100 µm und 450 µm, bevorzugt 200 µm bis 350 µm (gemessen in Luft, vor Aufsetzen des Messsystems auf die Haut, d.h. ohne Korrektur der Gewebebrechzahl), der Fokusdurchmesser auf einen Wert zwischen 6 µm und 10 µm, bevorzugt zwischen 7 µm und 9 µm, und die Apertur auf einen Wert zwischen 50 und 80 mrad (entsprechend dem Sinus des halben Öffnungswinkels des Aperturkegels an Luft, d.h. ohne Korrektur der Gewebebrechzahl) eingestellt.

Überraschend zeigt sich, dass durch diese Einstellung der Parameter für die homogenisierte Fluoreszenzanregung unerwünschte Effekte kompensiert und ausgeglichen werden können. Normalerweise wird die Anregungsstrahlung in einem Gewebe durch Streuung und Absorption geschwächt, so dass Gewebebereiche in größerer Tiefe schwächer zur Fluoreszenz angeregt werden als oberflächennahe Bereiche. Zudem wird das optische Signal aus oberflächennahen Bereichen bei seinem Weg vom Anregungsort zum Messsystem weniger geschwächt als optische Signale aus größeren Tiefen. Beides führt dazu, dass das gemessene optische Signal normalerweise stark übergewichtig von den oberflächennahen Bereichen bestimmt wird (bei Haut beispielsweise bedeutet dies, dass die Keratinschicht, die stark von Fremdstoffen wie Kosmetika beeinflusst wird und ohnehin von außen gut zu beobachten ist, das gewünschte optische Signal aus der Tiefe überstrahlt). Mit den hier gewählten Parametern zur Einstellung der Fokustiefe und -breite sowie der Apertur nimmt aufgrund der Zwei-Photonen-Anregung, bei welcher die Anregungswahrscheinlichkeit proportional zum Quadrat der Intensität wächst, die Anregungswahrscheinlichkeit in einem Maße zu wie die vorgenannten Tiefeneffekte die optischen Signale schwächen. Damit entsteht ein insgesamt weitgehend ausgeglichener Beitrag aller Gewebeschichten zum gemessenen optischen Signal.

Die Integrationstiefe für das optische Signal im Objekt (Gewebe) wird weitgehend dadurch begrenzt, dass der Zwei-Photonen-Effekt nach Erreichen der Fokustiefe nicht mehr wirkt. Durch optische Maßnahmen wie das Vorsehen einer Blende in der Kollektionsoptik (Kollektionseffizienzbegrenzung) kann dieser Abschneideeffekt noch verstärkt werden.

Als ein weiterer Vorteil zeigt sich, dass Schwankungen der Fokustiefe bei sonst konstanten Parametern der fokussierten Anregungsstrahlung das gemessene (über die Tiefe integrierte) optische Signal nur wenig beeinflussen. Die Messung ist somit vergleichsweise unempfindlich gegen Schwankungen der Ankoppelung des Messsystems an die Haut.

An dieser Stelle sei angemerkt, dass sich das Prinzip der fliegenden Optik wie auch der homogenisierten Fluoreszenzanregung durch geeignete Wahl von Blenden und Fokussierung vorteilhaft auch auf die konfokale Mikroskopie und die konfokale (Ein-Photonen-) Fluoreszenzmikroskopie anwenden lässt.

Der der Erfindung zugrunde liegende Gedanke soll nachfolgend anhand der in den Figuren dargestellten Ausführungsbeispiele näher erläutert werden. Es zeigen:
- Fig. 1: eine Übersichtsdarstellung einer Vorrichtung für die Mehr-Photonen-Fluoreszenzmikroskopie, aufweisend eine zentrale Steuer- und Verarbeitungseinheit, die über einen Tragarm mit einem an einen Patienten anzuordnendes Patientenmodul verbunden ist;
- Fig. 2: eine schematische Darstellung einer vertikalen (sagittalen) Schnittbildaufnahme;
- Fig. 3: eine schematische Übersichtsansicht einer Vorrichtung für die Mehr-Photonen-Fluoreszenzmikroskopie mit seinen Einzelkomponenten;
- Fig. 4: eine schematische Ansicht eines Patientenmoduls;
- Fig. 5: eine schematische Ansicht einer horizontal und vertikal beweglichen Optikeinheit des Patientenmoduls zur Aufnahme eines Schnittbildes;
- Fig. 6: eine schematische Ansicht eines Detektormoduls zur Detektion empfangener Signale;
- Fig. 7: eine graphische Darstellung zweier Beispielspektren;
- Fig. 8: eine diagrammatische Darstellung eines Verfahrens zur Signalverarbeitung;
- Fig. 9: eine Darstellung einer alternativen Ausführungsform eines Patientenmoduls;
- Fig. 10: eine Prinzipskizze einer homogenisierten Fluoreszenzanregung und
- Fig. 11A, 11B: graphische Darstellungen eines tiefenabhängigen Gewichtsfaktors in Abhängigkeit von der Gewebetiefe ohne Tiefenabschneidung (Fig. 11A) und mit Tiefenabschneidung (Fig. 11 B).

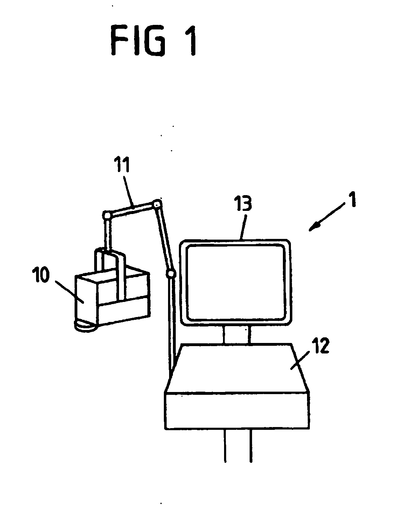

Fig. 1 zeigt in einer Übersichtsdarstellung eine Vorrichtung 1 für die Mehr-Photonen-Fluoreszenzmikroskopie zur Gewinnung von Informationen aus biologischem Gewebe, die eine Steuer- und Verarbeitungseinheit 12 aufweist, die über einen Tragarm 11 mit einem so genannten Patientenmodul 10 verbunden ist. Während die Steuer- und Verarbeitungseinheit 12 als zentrale, stationäre Einheit zum einen die Steuerung der Vorrichtung und zum anderen die Verarbeitung der empfangenen Signale übernimmt und Ergebnisse in geeigneter Weise über einen Monitor 13 ausgibt, ist das Patientenmodul 10 als in seiner Lage anpassbares, an einen Patienten anordbares Modul ausgebildet, das vorwiegend optische Komponenten zur Konditionierung, Übertragung und optischen Modifizierung einer einfallenden, von einer Lasereinheit der Steuer- und Verarbeitungseinheit 12 erzeugten und über eine optische Faser an das Patientenmodul 10 übertragenen Anregungsstrahlung einerseits und zur Aufnahme eines empfangenen optischen Signals andererseits aufweist.

Das Patientenmodul 10 stellt damit einen Messkopf dar, der über den mehrachsigen Tragarm 11 frei beweglich und präzise arretierbar ist, so dass nach genauer Positionierung des Patientenmoduls 10 relativ zu einem Patienten Aufnahmen mit mikroskopischer Auflösung angefertigt werden können. Das Patientenmodul 10 ist über faseroptische und elektrische Verbindungen flexibel mit der Steuer- und Verarbeitungseinheit 12 verbunden.

Zur Positionierung des Patientenmoduls 10 kann ein Fussschalter zur Steuerung einer motorischen Bewegung des Tragarms 11 vorgesehen sein.

Die Vorrichtung 1 ist vorteilhaft schwingungsgedämpft unter Minimierung der bewegten Masse ausgeführt. Das Gehäuse des Patientenmoduls 10 muss zur Vermeidung externer Störstrahlung lichtdicht sein.

Teil der Steuer- und Verarbeitungseinheit 12 ist auch ein Steuer-PC, über den Benutzereingabe und -ausgabegeräte (Maus, Tastatur, Joystick, Bildschirm) angeschlossen werden können.

Die Vorrichtung 1 lässt sich funktional in
- ein optisches System, bestehend beispielsweise aus einem Objektiv, einer Strahlformungs- und einer Kollektionsoptik, einer Anzahl von optischen Detektoren beispielsweise in Form von so genannten Photo Multiplier Tubes (PMTs), zugehörigen dichroitischen Filterelementen und Filtern, Quarzfasem zur Lichtübertragung, einem Ultrakurzpulslaser, einem Strahlabschwächer in Form eines Polarisators, einer Belichtungsvorrichtung zur Steuerung der Laserbelichtung und einer Realbildkamera mit zugehörigem Beleuchtungssystem,
- ein mechanisches System, bestehend beispielsweise aus dem Tragarm 11, Piezo-Linearmotoren mit Steuereinheiten und zugehörigem Tischsystem, einem Steppermotor für den Strahlabschwächer, einem Optikwechsler zum motorischen oder manuellen Wechseln zwischen Mikroskopieoptik und Realbildkamera und einem Patientenadapter zur Fixierung der Optik am Patienten,
- ein signalverarbeitendes System, bestehend beispielsweise aus Verstärkern, einer Elektronik zur Wandlung und Auswertung der empfangenen Signale und zur Daten-Speicherung, Photodioden zur Leistungskontrolle und Umgebungslichtüberwachung und zugehörigen Wandlern, sowie
- ein datenverarbeitendes System, bestehend beispielsweise aus einer graphischen Benutzeroberfläche mit Datenverwaltungsfunktionen und einem elektronischen Steuersystem, das alle Funktionen des optischen, des mechanischen und des signalverarbeitenden Systems koordiniert,
einteilen.

Die Vorrichtung 1 ist ausgebildet für die Mehr-Photonen-Fluoreszenzmikroskopie, insbesondere für die Zwei-Photonen-Mikroskopie. Die Vorrichtung 1 soll dabei als nichtinvasives System zur diagnostischen Unterstützung eines Arztes dienen, das mit Ausnutzung von Zweiphotonen-Prozessen insbesondere in vivo Querschnittsbilder der menschlichen Haut mit spektralen Zusatzinformationen liefert, aufgrund derer ein Arzt gegenüber einem reinen Oberflächenbild ergänzende Informationen zur Unterstützung seiner Diagnoseentscheidung erhält.

Grundsätzlich kann die Vorrichtung 1 für Diagnose unterstützende Anwendungen aller Entartungen der Haut genutzt werden, die sich in strukturellen Veränderungen des Gewebes widerspiegeln. Die Vorrichtung 1 kann dabei
- bildgebende Tiefeninformation mit mikroskopischer Auflösung aus epidermalen/dermalen Hautschichten bereitstellen,
- Sagittalschnitt-Darstellungen der Haut entsprechend den histopathologischen Dünnschnitten generieren,
- durch Verwendung unterschiedlicher spektraler Kanäle spektrale Informationen im Sinne einer ortsaufgelösten Wellenlängenband-Spektroskopie bereitstellen und
- Diagnostik unterstützende Funktionen, z.B. maschinelle Bewertungshilfen, liefern.

Die Vorrichtung 1 ist für Patienten jeden Alters mit Hautläsionen, die nicht allein durch klinische Begutachtung diagnostiziert, sondern beispielsweise biopsiert werden sollen, geeignet. Durch Anwendung der Vorrichtung 1 kann gegebenenfalls eine (invasive) Biopsie vermieden werden oder aber eine zielgerichtete Vorauswahl für Biopsien getroffen werden. Als Läsionen kommen insbesondere Hautpartien mit Verdacht auf Morbus Bowen, Basalzell-Karzinom, Plattenephitel-Karzinom bzw. aktinischer Keratose in Frage. Die Bedienung des Gerätes erfolgt dabei durch eingewiesenes Personal, das das Bildmaterial für den behandelnden Arzt bereitstellt und ggf. aufbereitet, oder durch den Arzt selbst.

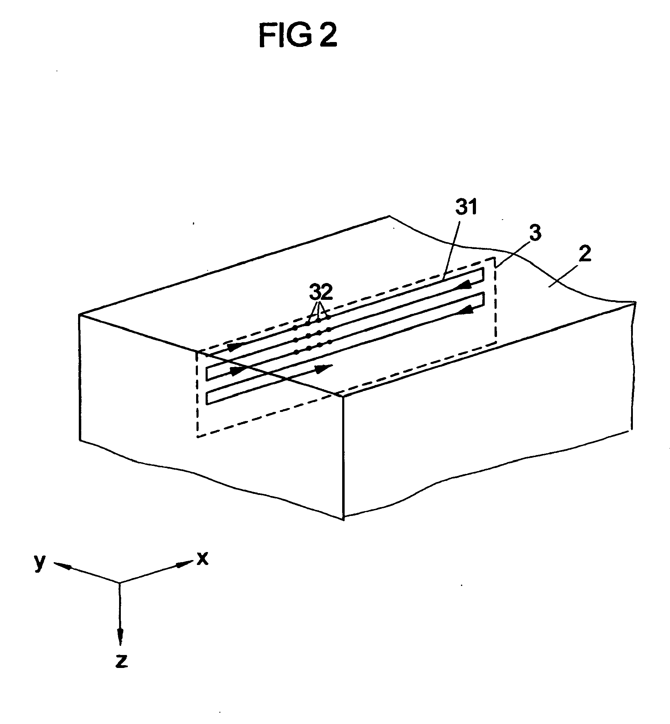

Mit der Vorrichtung 1 können insbesondere vertikale (sagittale) Schnittbilder durch die Haut eines Patienten aufgenommen werden. Dieses erfolgt, wie schematisch in Fig. 2 dargestellt ist, indem im Rahmen der Zwei-Photonen-Mikroskopie in einer sagittalen Bildebene 3 entlang einer Aufnahmelinie 31 das Hautgewebe an einzelnen Orten 32 angeregt wird. Der angeregte Ort 32 entspricht dabei jeweils einem Bereich um den Fokuspunkt einer Anregungsstrahlung mit einer Ausdehnung in X-Richtung (horizontal) von z.B. 0.5 µm und in Z-Richtung (vertikal) von z.B. 2 µm und wird selektiert durch Verschieben des Fokuspunktes entlang der Aufnahmelinie 31.

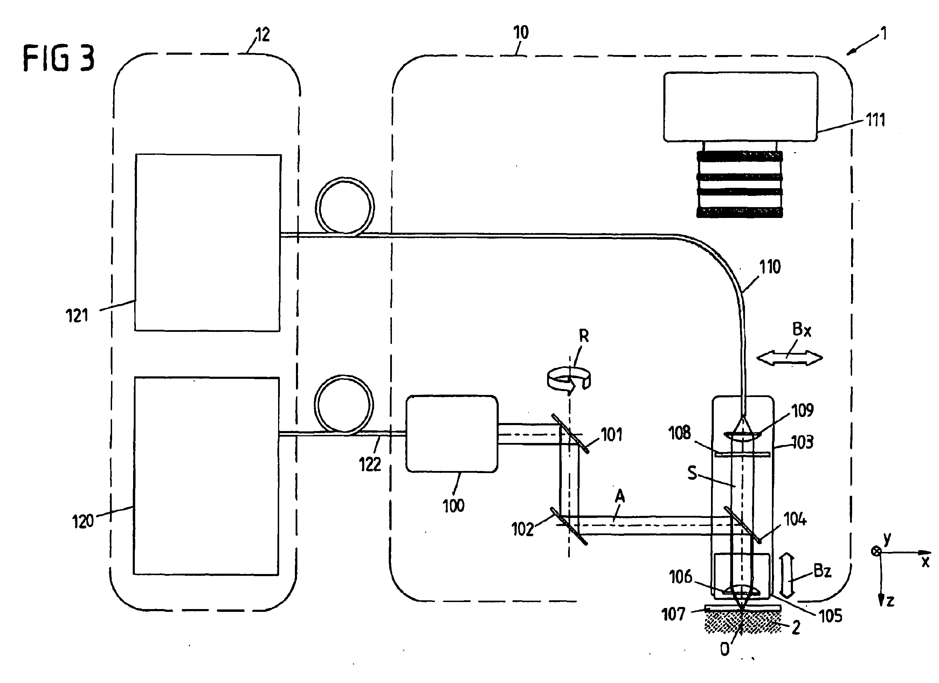

Bei der Vorrichtung 1 wird die Verschiebung des Fokuspunktes, wie in Fig. 3 bis 5 dargestellt ist und nachfolgend noch erläutert werden soll, durch eine in zumindest zwei Richtungen bewegliche Optikeinheit 103 des Patientenmoduls 10 erreicht.

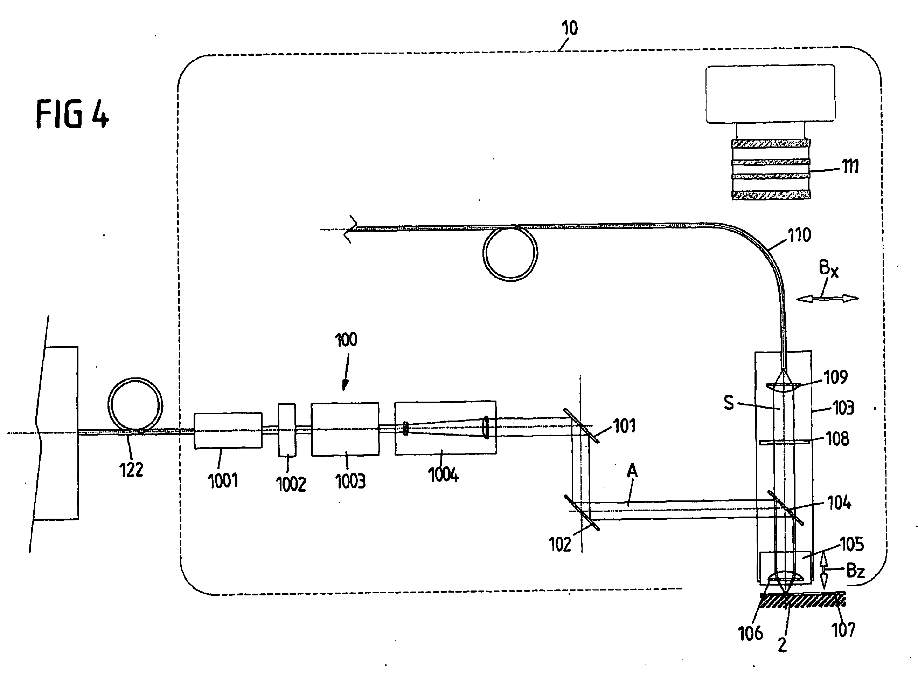

Fig. 3 zeigt in einer schematischen Übersichtsdarstellung eine Ausführungsform des optischen Systems der Vorrichtung 1 und Fig. 4 eine schematische Ansicht des optischen Aufbaus des Patientenmoduls 10.

Das optische System besteht im Wesentlichen aus einem kompakten Zwei-Photonen-Mikroskop, das eine Lasereinheit in Form eines Ultrakurzpulslasers 120 (fs-Faserlaser) aufweist, einem Detektionssystem in Form eines Detektormoduls 121 und einer Realbildkamera 111 samt Beleuchtung zur Aufnahme eines Realbildes.

Für die Zwei-Photonen-Mikroskopie erzeugt der in oder an der Steuer- und Verarbeitungseinheit 12 angeordnete Ultrakurzpulslaser 120 eine Anregungsstrahlung A mit einer Grundwellenlänge von 1560nm +/- 10nm, die über eine optische Faser 122 in Form einer Einmodenfaser dem Patientenmodul 10 zugeführt wird.

Im Patientenmodul 10 wird die Anregungsstrahlung A zunächst einer Vorrichtung zur Strahlkonditionierung 100 zugeleitet, in deren Rahmen, wie in Fig. 4 dargestellt ist, die Wellenlänge der Anregungsstrahlung A durch einen Frequenzverdoppler 1001 in Form eines geeigneten Kristalls auf 780 nm halbiert wird (die Frequenz also verdoppelt wird). Anschließend durchläuft die Anregungsstrahlung A einen Filter 1002, der die Grundwellenlänge (1560 nm), die dritte Harmonische (520 nm) und die vierte Harmonische (390 nm) herausfiltert und damit lediglich die frequenzverdoppelte Anregungsstrahlung A mit einer Wellenlänge von 780 nm passieren lässt. In einem Leistungseinsteller und - messer 1003 wird die Strahlungsleistung geregelt, wobei beispielsweise ein Abschwächer zur Abschwächung der Anregungsstrahlungsleistung vorgesehen sein kann. Der Abschwächer dient der Reduktion der Laserleistung nach der Frequenzverdopplung von einem Wert von beispielsweise ca. 100 mW auf einen Wert von 0% bis 100% der erlaubten Emissionsleistung (entsprechend der von der Optik auf das Objekt 2 abgestrahlten Leistung, beispielsweise max. 50mW) am Ort der Zwei-Photonen-Anregung. Der Abschwächer ist präzise und wiederholgenau über einen Motor einstellbar, wobei der Motor beispielsweise über eine Benutzerschnittstelle steuerbar ist. Mittels optischer Messung (beispielsweise über eine PIN-Photodiode) wird die IST-Leistung nach dem Abschwächer überwacht.

Schließlich durchläuft die Anregungsstrahlung A ein Teleskop 1004, das den Laserstrahl aufweitet und in geeigneter Weise formt.

Die Kenndaten des Ultrakurzpulslasers 120 können beispielsweise sein:
- Grundwellenlänge: 1560 nm +/- 50 nm;
- Wellenlänge nach Frequenzverdopplung: 780 nm +/-30 nm;
- Spektrale Breite (780 nm): 8,8 nm;
- Strahldurchmesser (780 nm): 1,3 mm;
- Strahldivergenz (780 nm): 3,8 mrad;
- M2 (780 nm): 1,07;
- Pulsfolgefrequenz: 100 MHz;
- Pulsdauer (780 nm): <150 fs;
- mittlere Leistung (780 nm): >100 mW.

Die Anregungsstrahlung A (mit einer Wellenlänge von 780 nm) kann in einer alternativen Lösung auch unmittelbar in der Steuer- und Verarbeitungseinheit 12 erzeugt und zum Patientenmodul 10 über eine geeignete optische Faser 122, insbesondere eine so genannte Photonic Fiber, übertragen werden.

Anstelle einer Faser 122 kann auch ein Spiegelgelenkarm zur flexiblen Übertragung eingesetzt werden.

Die Zwei-Photonen-Anregungsstrahlung A kann in einer weiteren alternativen Lösung unmittelbar im Patientenmodul 10 erzeugt werden, in die dann ein Laser integriert ist. Eine optische Faser zur Übertragung der Anregungsstrahlung A von der Steuer- und Verarbeitungseinheit 12 zum Patientenmodul 10 kann dann entfallen.

Als Strahlquelle kann in allen Fällen auch ein Laser in Form eines Femtosekundenlasers, wie beispielsweise ein Titan-Saphir-Laser, verwendet werden, der (anstelle der Strahlung bei 1560nm zur Erzeugung der Anregungsstrahlung bei 780nm) eine Anregungsstrahlung im Bereich 700 bis 900 nm, generiert, die dann ohne Frequenzverdopplung unmittelbar zur Anregung eingesetzt wird.

Nach erfolgter Strahlkonditionierung wird die Anregungsstrahlung A über Spiegel 101, 102 hin zu der Optikeinheit 103 gelenkt, dort über einen dichroitischen Spiegel 104 hin zu einem Objektiv 105 umgelenkt, durch eine asphärische Linse 106 fokussiert und auf ein Objekt 2, beispielsweise die Haut eines Patienten gestrahlt.

Das Patientenmodul 10 weist einen für die Anregungsstrahlung A durchlässigen Kontaktabschnitt 107 in Form einer Glasscheibe auf, die mit der Haut beispielsweise unter Verwendung eines Immersionsfluids zur Verbesserung der mikroskopischen Auflösung ortsfest in Kontakt steht.

Zur Einstellung des Ortes des Fokus ist die Optikeinheit 103 einstellbar. Die Optikeinheit 103 ist dabei zur lateralen Bewegung des Fokuspunktes relativ zu dem Kontaktabschnitt 107 zumindest in X-Richtung (entsprechend einer lateralen Bewegung BX) beweglich, vorteilhafterweise auch in Y-Richtung, also zweidimensional entlang der Oberfläche des Objektes 2. Gleichzeitig ist das Objektiv 105 der Optikeinheit 103 in Z-Richtung (entsprechend einer axialen Bewegung BZ) verstellbar, um auf diese Weise den Fokuspunkt auch in Z-Richtung vertikal zu bewegen (alternativ ist auch denkbar, die gesamte Optikeinheit 103 in Z-Richtung beweglich auszubilden). Durch Bewegung des Fokuspunktes innerhalb des Objekts 2 können Schnittbilder erzeugt werden, wobei die laterale Beweglichkeit der Optikeinheit 103 die Generierung von Schnittbilder mit großen lateralen Kantenlängen von beispielsweise mehreren mm oder auch cm möglich macht.

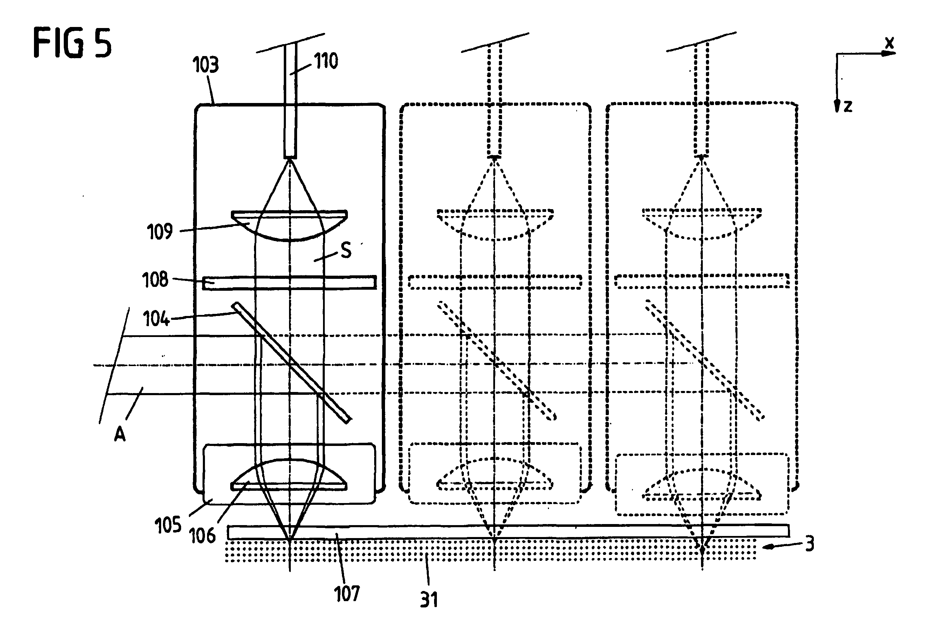

Fig. 5 zeigt schematisch die Bewegung der Optikeinheit 103 während des Aufnahmevorgangs eines vertikalen (sagittalen) Schnittbildes. Zur Aufnahme von Signalen an unterschiedlichen, lateral versetzen Orten 32 wird die Optikeinheit entlang der Aufnahmelinie 31 (siehe Fig. 2) zunächst kontinuierlich entlang der X-Richtung verfahren und damit horizontal relativ zu dem Objekt 2 (und dem ortsfest am Objekt 2 angeordneten Kontaktabschnitt 107 in Form der Glasscheibe des Patientenmoduls 10) bewegt. Bei der Bewegung der Optikeinheit 103 werden unterschiedliche Orte 32 durch eine ortsabhängig getriggerte Belichtung mit der Anregungsstrahlung A angeregt, und das angeregte Signal wird aufgenommen. Auf diese Weise wird eine Anzahl von Bildpunkten (beispielsweise mehrere hundert oder auch tausend) in einer Zeile aufgenommen. Ist das Ende der Zeile erreicht, wird das Objektiv 105 der Optikeinheit 103 in Z-Richtung verstellt und damit der Fokus in Z-Richtung bewegt. Die Optikeinheit 103 wird dann entlang der X-Richtung zurück verfahren, die nächste Zeile wird aufgenommen und so weiter, bis schließlich das gesamte Bild aufgenommen worden ist.

Diese Reihenfolge der Abtastung (erst lateral, dann in die Tiefe) kann selbstverständlich auch in umgekehrter Abfolge (erst in die Tiefe, dann lateral) geschehen.

Dabei kann die oberflächennormale Abtastung ("Z-Abtastung") durch eine vertikal schwingende Aufhängung des Objektivs 105 bewirkt werden, die periodisch angeregt oder angetrieben wird und so die Z-Bewegung ausführt. Infrage kommen für die schwingende Aufhängung insbesondere Blattfedern, Blattfedergelenke, Ringfedern oder Luftfedern, die piezoelektrisch, elektromagnetisch oder motorgetrieben über ein mechanisches Nocken- oder Exzentergetriebe angetrieben werden. Die Lateralbewegung der Optikeinheit 103 erfolgt dann kontinuierlich in nur einem Durchgang pro Schnittbild, d.h. entsprechend langsamer. Der Vorteil dieser Anordnung liegt darin, dass das Objektiv 105 eine deutlich geringere Masse besitzt als die Optikeinheit 103 als Ganzes, deshalb mit geringeren Massenkräften schnell bewegt werden kann und somit weniger Vibrationen der Vorrichtung 1 erzeugt.

Weiterhin kann die Abtastung nicht nur zweidimensional ("2D"), sondern auch dreidimensional ("3D") erfolgen, indem in beliebiger Reihenfolge nacheinander zwei laterale (X und Y) und ein Tiefen-Scan (Z) vorgenommen werden.

Die bewegliche Optikeinheit 103 des Patientenmoduls 10 ermöglicht eine motorisierte Bewegung der abbildenden Optik über das Objekt 2, wobei Richtung (0°-180°), laterale Lage und laterale Länge durch den Nutzer frei wählbar sind. Die Bewegung der Optikeinheit 103 in der Ebene parallel zur Objektoberfläche kann beispielsweise durch zwei Piezo-Linearmotoren erfolgen, die mechanisch gekoppelt und koordiniert bewegt werden. Alternativ zu einer koordinierten Motorenbewegung kann auch nur ein Linearmotor für eine eindimensionale Bewegung entlang der Hautoberfläche vorgesehen sein, wobei zur Festlegung der Scanrichtung das gesamte Patientenmodul 10 mitsamt der Optikeinheit 103 durch den Anwender manuell gedreht und ausgerichtet wird, um die Scanrichtung zu bestimmen.

Eine der Lateralbewegungen kann auch (im Sinne einer Rasterung in Polarkoordinaten) als Drehbewegung um die in Fig. 3 dargestellte Rotationsachse R geschehen. Ein erster Motor bewegt dann die Optikeinheit 103 linear (in radialer Richtung), während ein zweiter Motor die Optikeinheit 103 um die Rotationsachse R rotiert.

Die Auflösung des aufgenommenen Bildes wird durch die Größe seiner Bildpunkte (Pixel) bestimmt. Im Falle der Zwei-Photonen-Fluoreszenz sind diese durch die Fokusgröße der Anregungsstrahlung A festgelegt, wobei vorteilhaft eine Fokusgröße von 0.5 µm in lateraler Richtung (X-Richtung) und 2 µm in axialer Richtung (Z-Richtung) eingesetzt wird, um eine zelluläre Auflösung zu erreichen. Um die Masse der zu bewegenden Optikeinheit 103 so gering wie möglich zu halten, wird zur Fokussierung eine einzelne asphärische Linse 106 verwendet, was möglich ist dadurch, dass die optische Achse O der auf das Objekt 2 gestrahlten Anregungsstrahlung A in ihrer Winkellage bei Aufnahme eines Bildes nicht verändert wird (im Gegensatz beispielsweise zur Verwendung von Dreh- und Kippspiegeln) und daher nur im unmittelbaren Bereich der optischen Achse O optimale Fokussiereigenschaften benötigt werden. Die asphärische Linse 106 kann beispielsweise eine geometrisch-optische Brennweite von f = 8 mm, einen Arbeitsabstand (definiert als die fokusseitige Schnittweite, d.h. der Abstand des Fokuspunktes von der nächstgelegenen optischen Fläche der Linse) von 6 mm und eine numerische Apertur von NA = 0.55 aufweisen.

Mittels der Anregungsstrahlung A wird Sekundärstrahlung an einzelnen Orten entsprechend dem Fokuspunkt der Anregungsstrahlung A im Objekt 2 angeregt. Die Sekundärstrahlung kann dabei einerseits aus einer Fluoreszenzstrahlung, erzeugt durch körpereigene oder körperfremde Stoffe, und andererseits aus in dem Objekt an Strukturen erzeugten Oberwellen, insbesondere der zweiten Harmonischen (SHG: second harmonic generation), bestehen. Die Sekundärstrahlung wird als Signal S von der Optikeinheit 103 aufgenommen und über die Linse 106, den dichroitischen Spiegel 104, einen Sperrfilter 108 zur Unterdrückung der Anregungsstrahlung A und eine Linse 109 in eine zweite optische Faser 110 eingekoppelt und an das Detektormodul 121 der Steuer- und Verarbeitungseinheit 12 übertragen. Die Linsen 106 und 109 können bevorzugt als Asphäre, alternativ aber auch in jeweils unabhängiger Wahl als sphärische Einzellinse oder Linsengruppe, (z.B. als Achromat oder Mikroskopobjektiv) oder als abbildende Spiegelanordnung ausgeführt sein.

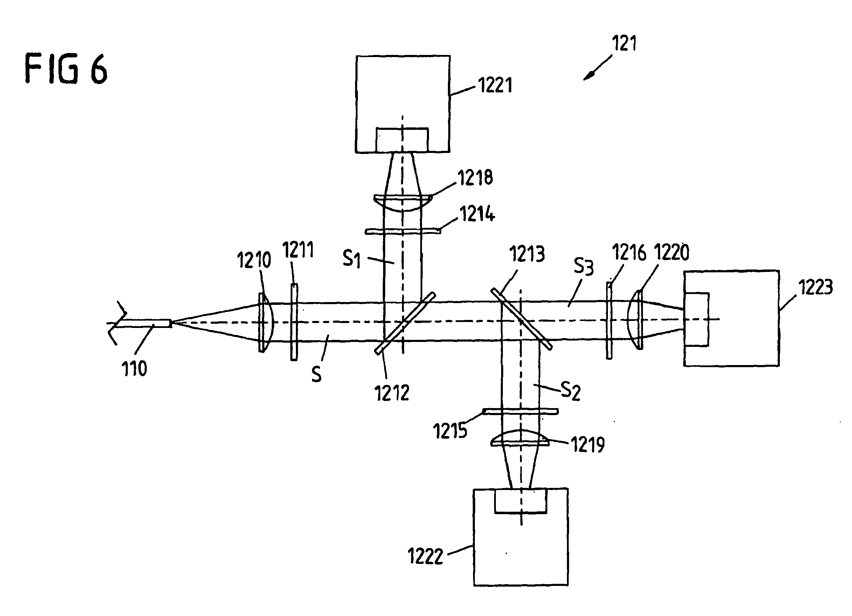

Die empfangenen Signale werden an das Detektormodul 121 der Steuer- und Verarbeitungseinheit 12 übertragen (siehe Fig. 3). Eine Detailansicht einer Ausführungsform des optischen Aufbaus des Detektormoduls 121 ist in Fig. 6 dargestellt. Das Detektormodul 121 ist dabei dreikanalig aufgebaut und weist drei Detektoren in Form von Sekundärelektronenvervielfachern 1221, 1222, 1223 (Photo Multiplier Tube, PMT) auf. Das dem Detektormodul 121 über die optische Faser 110 zugeführte Signal S wird durch eine asphärische Linse 1210 gebündelt, durch einen Sperrfilter 1211 in Form eines Kurzpassfilters zur weiteren Unterdrückung reflektierter oder gestreuter Anregungsstrahlung A geführt, durch dichroitische Filterelemente 1212, 1213 in drei Signalanteile S1, S2, S3 in unterschiedlichen Wellenlängenbändern zerlegt und über Sperrfilter 1214, 1215, 1216 und Linsen 1218, 1219, 1220 den Detektoren 1221, 1222, 1223 zugeführt.

Bei einem vier- oder mehrkanaligen Aufbau können entsprechend der Anzahl der Kanäle auch mehr Filter, Linsen und Detektoren vorgesehen sein.

Als Detektoren 1221, 1222, 1223 werden PMTs mit geeigneter spektraler Empfindlichkeit (Photokathoden) verwendet, die über die als Strahlteiler dienenden dichroitischen Filterelemente 1212, 1213 Teilbänder des sichtbaren Spektrums aus dem über die Faser 110 zugeführten Fluoreszenzlicht erhalten. Das gesamte System befindet sich in einem vollkommen lichtdichten und innen geschwärzten Gehäuse, um störende Reflexe und Streulicht zu unterdrücken. Die von den PMTs erzeugten elektrischen Signale werden zunächst verstärkt - wobei die Verstärker in der Umgebung der PMTs angeordnet sein sollten, um elektromagnetische Einstreuungen zu minimieren - und dann digitalisiert.

Als Messgröße für die Detektion dienen die Ladungen pro Pixel aus den unterschiedlichen Kanälen, die pro Detektor 1221, 1222, 1223 aus der Integration des Photostroms über eine Pixelintegrationsdauer gewonnen werden. Aus diesen Signalen wird mit Hilfe von geeigneten Parametern (z.B. Kalibrierungswerten pro Kanal, Helligkeits- und Kontrastkorrektur, Falschfarbenfunktion und dergleichen) ein Fluoreszenzbild erstellt. Die Ladungen werden hierzu in einem vorgebbaren Pixel-Takt z.B. durch Integration eines Stroms durch die Detektoren 1221, 1222, 1223 in eine Spannung gewandelt, und diese wird mit z.B. 12 bit digitalisiert.

PMTs erzeugen bei Lichteinfall kleine Ströme im Bereich von nA bis µA, die entsprechend weiterverarbeitet werden müssen. Jedem PMT ist ein Stromverstärker nachgeschaltet, der beispielsweise eine Bandbreite von 200 kHz bis 8 MHz, einen Verstärkungsfaktor ≤ 10⁵ und eine Eingangsimpedanz gleich der Impedanz des PMT (50 Ohm) aufweist und dabei driftstabil und rauscharm ausgeführt ist. Jedem Verstärker ist eine Integrationsschaltung nachgeschaltet, die extern (durch einen Encoder-Takt, der aus der bewegten Optik abgeleitet wird) getriggert wird und das Stromsignal des zugeordneten PMTs in einen Spannungswert wandelt. Diese Spannung wird mittels eines Analog-Digital-Wandlers in einen 12-bit-Wert gewandelt und in einem Pufferspeicher abgelegt, von dem die Daten an eine Rechnerschnittstelle zur Weiterverarbeitung gesendet werden.

Die PMTs oder SiPMTs können bei niedrigen Signal-Photonenflussraten alternativ auch im Single-Photon-Counting-Betrieb verwendet werden. Als digitale Ausgangssignale entstehen in diesem Fall pro Pixel und Kanal je eine registrierte Ereigniszahl, der "Photonen-Count".

Bei der Aufnahme eines Mikroskopie-Schnittbildes wird die Optikeinheit 103 (siehe Fig. 3 und Fig. 5) mit einer konstanten Lineargeschwindigkeit kontinuierlich entlang der X-Richtung verfahren. Dabei wird der Messtakt durch einen ortsausgelösten Trigger bestimmt, nämlich den Encodertakt des Linearmotors. Da mit mikroskopischer Genauigkeit (Auflösung < 1µm) gemessen werden soll, sind die Anforderungen an die Genauigkeit des Rastervorgangs für die bewegte Optikeinheit 103 hoch. Wird der Integrationsvorgang der Detektoren 1221, 1222, 1223 durch den Encodertakt des Linearmotors gesteuert, wirken sich Schwankungen der Bewegungsgeschwindigkeit nicht als Bildverzerrungen aus, wobei Abweichungen bei Start- bzw. Endpositionen der einzelnen Zeilen die Weite eines Pixels, also z.B. 0.5 µm, nicht überschreiten sollten. Die Integration für alle Kanäle muss exakt zeitgleich stattfinden, um ortsgleiche Daten in den einzelnen Kanälen zu erhalten.

Zur Steuerung der Belichtung des Objekts mit der Anregungsstrahlung wird ein so genannter Shutter eingesetzt, der als lichtundurchlässiges Element ausgebildet ist und zur Abschirmung und Unterbrechung der Anregungsstrahlung in den Strahlengang der Anregungsstrahlung geklappt wird. Der Shutter wird nur während einer Aufnahme geöffnet und ist insbesondere während der Systemprüfung, bei einem Not-Aus, bei fehlendem Patientenkontakt, solange keine Aufnahme durchgeführt wird und in den Umkehrpunkten einer Aufnahmelinie zur Minimierung der Laser-Einstrahlung in die Haut des Patienten geschlossen. Der Shutter kann durch eine Steuerungssoftware gesteuert werden, wobei in einem Fehlerfall eine automatische und Software-unabhängige Schließung vorgesehen sein kann. Der Shutter wird zur Belichtung der einzelnen Orte des Objekts zur Erzeugung der einzelnen Pixelsignale während einer Aufnahme mit vergleichsweise hoher Taktrate (im Bereich weniger ms) geschaltet, wobei gegebenenfalls ein Haupt- und ein Mess-Shutter vorgesehen sein können, von denen der Haupt-Shutter bei einer Aufnahme grundsätzlich geöffnet ist und der Mess-Shutter ausgelöst durch den Trigger geschaltet wird.

Bei dem Aufbau gemäß Fig. 6 sind drei Kanäle zur Erfassung von drei spektralen Bändern vorgesehen. Dazu können gehören:
- ein SHG-Kanal in einem Wellenlängenband von 390 nm ± 5 nm,
- ein erster kurzwelliger Kanal in einem Wellenlängenband von 450 nm ± 50 nm und
- ein zweiter kurzwelliger Kanal in einem Wellenlängenband von 550 nm ± 50 nm.

Dabei soll der SHG-Kanal ausschließlich die schmalbandige, nicht-resonant erzeugte Gewebe-SHG detektieren, während die zwei kurzwelligen Kanäle das Auto-Fluoreszenzsignal mit der Möglichkeit detektieren, eine ortsaufgelöste spektrale Verhältnisbildung zu ermitteln.

Zusätzlich kann in einem erweiterten Aufbau ein vierter Kanal in einem Wellenlängenband von 625 nm ± 25 nm vorgesehen sein, der zur Erfassung von Fluoreszenzsignalen bestimmt ist, die durch Marker wie PP IX oder ALA bedingt sind.

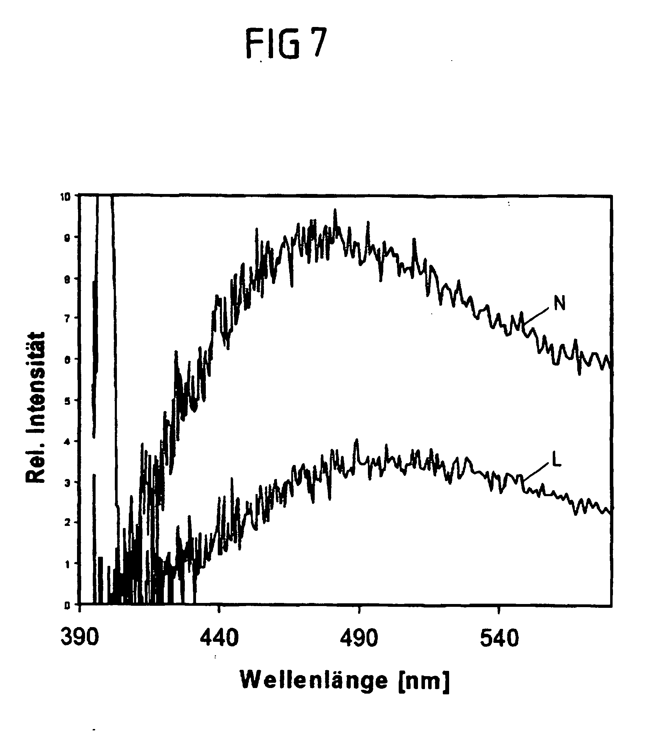

Zwei Beispielspektren sind in Fig. 7 dargestellt, einmal für ein Spektrum N aus einer Normalhaut und einmal für ein Spektrum L aus einer Läsion, also einer pathologisch veränderten Haut. Deutlich sichtbar in den beiden Spektren L, N ist beispielsweise eine durch die Generierung der zweiten Harmonischen (SHG) bedingte Signalspitze um 390 nm sowie ein Spektralverlauf mit einem ausgeprägten Maximum bei ca. 480 nm (N) bzw. 500 nm (L). Die Signalspitze bei 390 nm kann durch den SHG-Kanal aufgenommen werden, während durch Verhältnisbildung der Signale der zwei kurzwelligen Kanäle auf die Position des Maximums in einem Spektrum L, N zurück geschlossen werden kann.

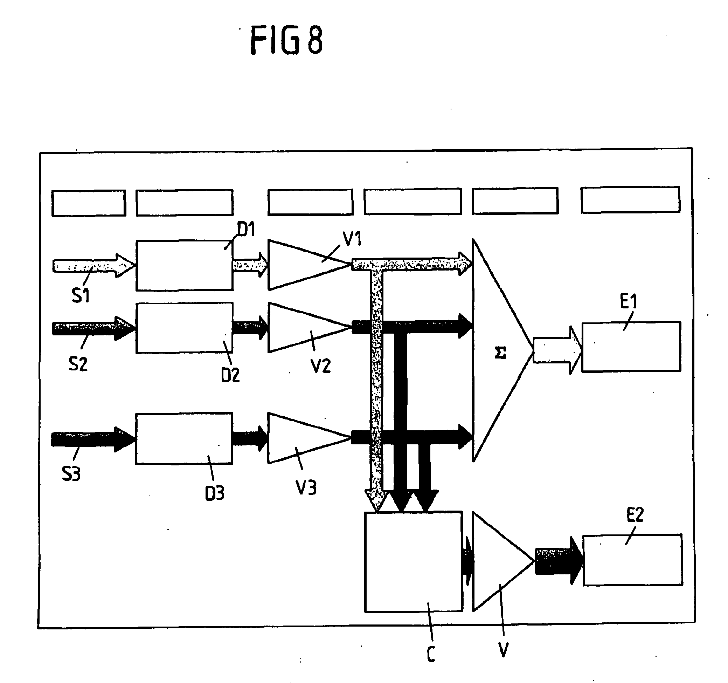

In Fig. 8 ist ein Verarbeitungsschema unterschiedlicher Signalanteile S1, S2, S3 bei mehrkanaliger Ausgestaltung des Detektormoduls 121 dargestellt. Die unterschiedlichen Signalanteile S1, S2, S3 werden dabei durch jeweils einen Detektor D1, D2, D3 (beispielsweise einen PMT, vgl. Fig. 6) detektiert und in jeweils ein elektronisches Datensignal gewandelt, das anschließend durch Verstärker V1, V2, V3 verstärkt wird. Die so erhaltenen elektronischen Signale können nun zur Informationsgewinnung weiterbearbeitet werden. Beispielsweise können durch Summenbildung Σ die Signalanteile S1, S2, S3 zu einem Gesamtsignal kombiniert werden, aus dem eine Helligkeitsinformation gewonnen wird. Das Ergebnisbild E1 kann beispielsweise als Schwarzweiß-Bild auf einem Monitor angezeigt werden und einem Nutzer eine Aussage darüber geben, welche Signalintensitätsverteilung sich in dem betrachteten Gewebe ergibt. Gleichzeitig können die einzelnen Signalanteile S1, S2, S3 nach der Verstärkung auch getrennt weiterverarbeitet werden, um Ergebnisbilder E2 zu erzeugen, die beispielsweise Informationen über das Vorhandensein eines Fluophors an bestimmten Orten im Gewebe liefern und dem Helligkeitsbild E1 in Falschfarben überlagert werden können. Es ergibt sich ein Bild, bei dem die Helligkeitsinformation in Schwarzweiß und zusätzliche spektrale Informationen aus den einzelnen Signalbändern in Falschfarben angezeigt werden.

Die genannten kombinierten Bildinformationen (Ergebnisbilder E1, E2 etc.) können aus den Signalen S1, S2 etc. wie dargestellt analog erzeugt werden. Alternativ kann auch gleich nach den Verstärkern V1, V2 etc. analog-digital gewandelt werden, um anschließend die genannte Ergebnisbilder E1, E2 durch digitale Signalverarbeitung oder Berechnung zu gewinnen.

Unterschiedliche Signale aus den unterschiedlichen Bändern können dem Helligkeitsbild E1 in unterschiedlichen Farben überlagert werden. Beispielsweise kann das dem PP IX entsprechende Signal in rot und das der zweiten Harmonischen (SHG) entsprechende Signal in blau angezeigt werden, wobei vorgesehen sein kann, dass ein Nutzer zur problembezogenen Optimierung der Darstellung und zur Darstellung gezielter Diagnose-Informationen mittels eines Eingabeinstruments wie eines Joysticks, eines Schiebereglers oder gegebenenfalls mittels Sprachsteuerung den Signalpegel der einzelnen Signale zur Überlagerung einstellen kann.

Gegebenenfalls kann auch eine logarithmische Helligkeitskorrektur oder eine automatische Kontrastoptimierung vorgenommen werden.

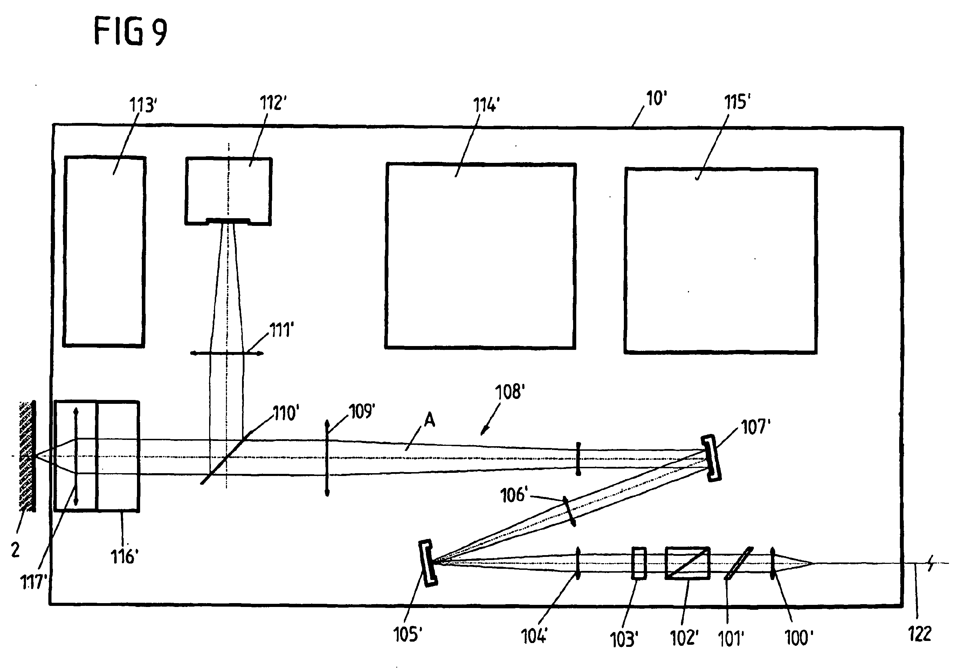

Fig. 9 zeigt eine Ausgestaltung eines Patientenmoduls 10', bei der ein Zwei-Achsen-Kippspiegel 107' zur Verstellung des Fokuspunktes der Anregungsstrahlung A und damit des Ortes der Anregung verwendet wird. Die über die optische Faser 122 zugeleitete Anregungsstrahlung A wird dabei zunächst durch eine Linse 100' kollimiert, durch einen Frequenzverdoppler 101' in der Wellenlänge halbiert (beispielsweise von 1560 nm auf 780 nm), durch einen Polarisator 102' und ein Lambda-Viertelplättchen 103' zirkular polarisiert (die von der optischen Faser 122 übertragene Anregungsstrahlung A ist ursprünglich linear polarisiert), durch eine Linse 104' auf einen ersten adaptiven Spiegel 105' und von diesem über eine Linse 106' auf den Zwei-Achsen-Kippspiegel 107' gelenkt.

Die Spiegel 105', 107' können beispielsweise als MEMS-Bauelemente (MEMS: Mikroelektromechanische Systeme) hergestellt sein. Der erste Spiegel 105' dient dabei zur Variation der Wellenfront, während der zweite Spiegel 107' durch Zwei-Achsen-Verkippung die Anregungsstrahlung A zur räumlichen Bewegung des Fokuspunktes ablenkt.

Die so abgelenkte Anregungsstrahlung A wird durch ein Teleskop zur Strahlaufweitung 108', eine Linse 109', einen dichroitischen Strahlteiler 110', ein Objektiv 116' mit einer asphärischen Linse 117' auf ein Objekt 2, beispielsweise die Haut eines Patienten, gerichtet und regt das Gewebe zur Emission einer Sekundärstrahlung an, die wiederum über das Objektiv 116' und den Strahlteiler 110' über eine Linse 111' hin zu einem Detektor 112' geleitet wird, der die Sekundärstrahlung als optisches Signal empfängt und in elektronische Datensignale wandelt.

Zur Auswertung der Datensignale sowie zur Steuerung der einzelnen Baugruppen weist das Patientenmodul 10' eine Treiberelektronik 113' (insbesondere zur Steuerung einer vertikalen Bewegung des Objektivs 116'), eine Detektorelektronik 114' zur Steuerung des Detektors 112' und zur Weiterverarbeitung der empfangenen Signale und eine Steuerelektronik 115' zur Einstellung der Spiegel 105', 107' auf.

Als eine zusätzliche Maßnahme bei sämtlichen vorangehend beschriebenen Ausführungsbeispielen der Vorrichtung 1 kann der Fokus der Zwei-Photonen-Anregungsstrahlung A durch geeignete Mittel schnell über eine geringe Auslenkung um seine Mittellage bewegt ("gewobbelt") werden, damit die Fluoreszenzanregung während der Aufnahmezeit (= Integrationszeit) über ein geeignetes Volumen gemittelt werden kann. Das so erhaltene Aufnahmevolumen ("Intergrationsvolumen"), aus dem die Zwei-Photonen-angeregten Signale S stammen, ist größer als das dem Fokuspunkt entsprechende Volumen und liefert ein für die Spektroskopie ausreichend starkes, auswertbares Signal. Neben einer Homogenisierung des Signals S wird damit der Zweck erreicht, die Probe oder das untersuchte Gewebe nicht durch lang andauerndes Bestrahlen desselben mikroskopischen Fokusvolumens unnötig zu belasten.

In besonders vorteilhafter Weise kann diese Mikrobewegung des Fokus in einer solchen Weise ausgeführt werden, dass die Anregungsvolumina, d.h. diejenigen Bereiche der Foki, deren Intensität für die Mehrphotonenanregung ausreicht, direkt aufeinander folgender Laserpulse nicht überlappen. Der Vorteil einer solchen Verfahrensweise liegt darin, dass das Risiko einer optischen Schädigung von Gewebe signifikant verringert wird. Bei Überlapp trifft der Folgepuls auf bereits vom Vorpuls optisch angeregte Gewebebereiche. Da der zeitliche Pulsabstand bei den verwendeten Ultrakurzpulslasern in der Größenordnung von 10 ns liegt, also in der Größenordnung der Fluoreszenzabklingzeiten, besteht eine nicht vernachlässigbare Wahrscheinlichkeit dafür, primär angeregte Moleküle im Gewebe in einen noch höheren Energiezustand zu transformieren, der dann zu bleibenden Veränderungen der Moleküle führt.

Für die schnelle Mikrobewegung ("Wobbeln") der Anregungsstrahlung A für die Zwei-Photonen-Spektroskopie kann beispielsweise ein schwingendes oder rotierendes optisches Bauteil, z.B. eine Linse, ein Spiegel, ein Prisma oder dergleichen verwendet werden.

Eine vorteilhafte Ausführungsform stellt ein Taumelspiegel dar, d.h. ein Spiegel, der auf einer motorisch zu einer schnellen Drehbewegung angetriebenen Rotationsachse so montiert ist, dass zwischen der Rotationsachse und der Spiegelnormalen ein kleiner Winkel (beispielsweise 0,5° bis 2,5°) besteht. Der reflektierte Strahl rotiert demnach schnell um seine Achse, was zu einer kleinen Kreisbewegung des Fokus und damit zu einer überlappfreien Fokusbewegung führt.

Eine weitere vorteilhafte Ausführung ist ein in zwei Achsen schwingender Spiegel, insbesondere ein MEMS, bei dem die Schwingungsfrequenzen der beiden senkrecht zueinander liegenden Hauptschwingungsachsen in einem Verhältnis zueinander stehen, das einer rationalen Zahl als Bruch zweier Primzahlen entspricht. Wird diese zweiachsige Schwingung des Spiegels durch einen z.B. elektromagnetischen oder elektrostatischen Antrieb angeregt, so vollführt der reflektierte Strahl und damit der Laserfokus Lissajous-Figuren, bei denen im Schwingungsumkehrpunkt der einen Schwingungsachse die andere nicht die Geschwindigkeit Null, sondern einen endlichen Wert besitzt. Somit gibt es bei dieser Bewegung niemals einen Stillstand des Fokus, der ja zu dem unerwünschten Überlapp der Anregungsvolumina von Folgepulsen führen würde.

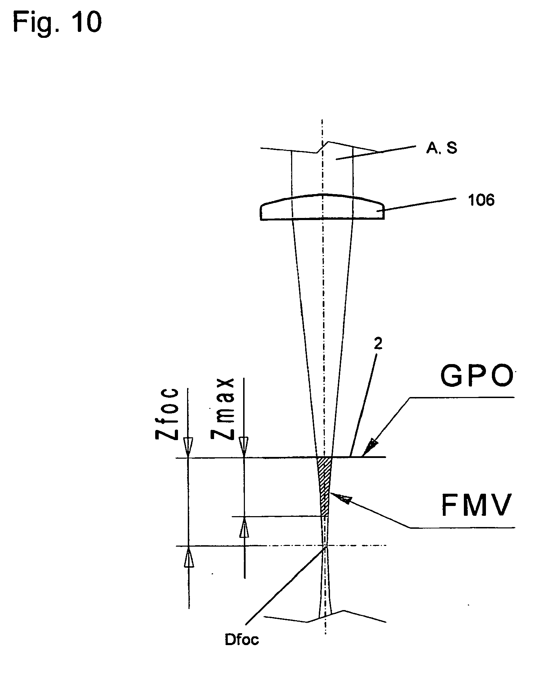

Fig. 10 zeigt in einer (nicht maßstäblichen) Prinzipdarstellung eine homogenisierte Fluoreszenzanregung, bei der ein lateral begrenzter, jedoch axial (vertikal) ausgedehnter Bereich ("FMV" = Fluoreszenzmessvolumen) des Objekts 2 so angeregt wird, dass in axialer Richtung das Objekt 2 weitestgehend gleichgewichtig zumindest über einen bestimmten Tiefenbereich zum optischen Signal S beiträgt. Dieses optische Signal S wird aufgenommenen und integriert und kann als einzelner Messwert, als Spektrum oder mehrkanalig mit separaten Spektralbanden ("Bandenspektroskopie") ausgewertet und weiterverarbeitet werden.

Zur Aufnahme wird die Optikeinheit 103 (siehe z.B. Fig. 3) ausschließlich in horizontaler (lateraler) Richtung (in X- oder in X- und Y-Richtung) zur Oberfläche des Objekts 2 bewegt (eine Bewegung in vertikaler Richtung (Z) ist nicht erforderlich weil das optische Signal S über die Tiefe integriert wird).

Um eine homogenisierte Fluoreszenzanregung in einem als Fluoreszenzmessvolumen FMV bezeichneten Bereich zu erhalten, werden die Parameter für die Apertur des Objektivs 106, der Fokusdurchmesser Dfoc und die Fokustiefe Zfoc auf Werte innerhalb bestimmter Parameterbereiche eingestellt. So wird eine vergleichsweise kleine Apertur zwischen 50 und 80 mrad (entsprechend dem Sinus des halben Öffnungswinkels des Aperturkegels an Luft, d.h. ohne Korrektur der Gewebebrechzahl) gewählt. Die Fokustiefe Zfoc wird vorteilhafterweise auf einen Wert zwischen 100 µm und 450 µm, bevorzugt 200 µm bis 350 µm (gemessen in Luft, vor Aufsetzen des Messsystems auf die Haut, d.h. ohne Korrektur der Gewebebrechzahl) und der Fokusdurchmesser Dfoc auf einen Wert zwischen 6 µm und 10 µm, bevorzugt zwischen 7 µm und 9 µm, eingestellt.

Das Fluoreszenzmessvolumen FMV erstreckt sich von der Gewebe- oder Probenoberfläche GPO (Objekt 2) axial in die Tiefe bis zu einer Integrationstiefe Zmax. Die Fokustiefe Zfoc ist größer (tiefer) als die Integrationstiefe Zmax.

Normalerweise wird die Anregungsstrahlung A in einem Gewebe (Objekt 2) durch Streuung und Absorption geschwächt, so dass Gewebebereiche in größerer Tiefe schwächer zur Fluoreszenz angeregt werden als oberflächennahe Bereiche. Zudem wird das optische Signal S aus oberflächennahen Bereichen bei seinem Weg vom Anregungsort zum Messsystem weniger geschwächt als optische Signale S aus größeren Tiefen. Beides führt dazu, dass das gemessene optische Signal S normalerweise stark übergewichtig von den oberflächennahen Bereichen bestimmt wird.

Überraschend hat sich gezeigt, dass mit den hier gewählten Parametern zur Einstellung der Fokustiefe Zfoc und der Fokusbreite Dfoc sowie der Apertur ein insgesamt weitgehend ausgeglichener Beitrag aller Gewebeschichten innerhalb des Fluoreszenzmessvolumens FMV mit einer Integrationstiefe Zmax zum gemessenen optischen Signal S erhalten wird. Dies wird dadurch bewirkt, dass die Skalierungsregel der Zwei-Photonen-Anregung mit der Intensität (bei welcher die Anregungswahrscheinlichkeit proportional zum Quadrat der Intensität wächst) die Schwächung der Anregungsstrahlung A und des optischen Signals S im Gewebe weitestgehend kompensiert.

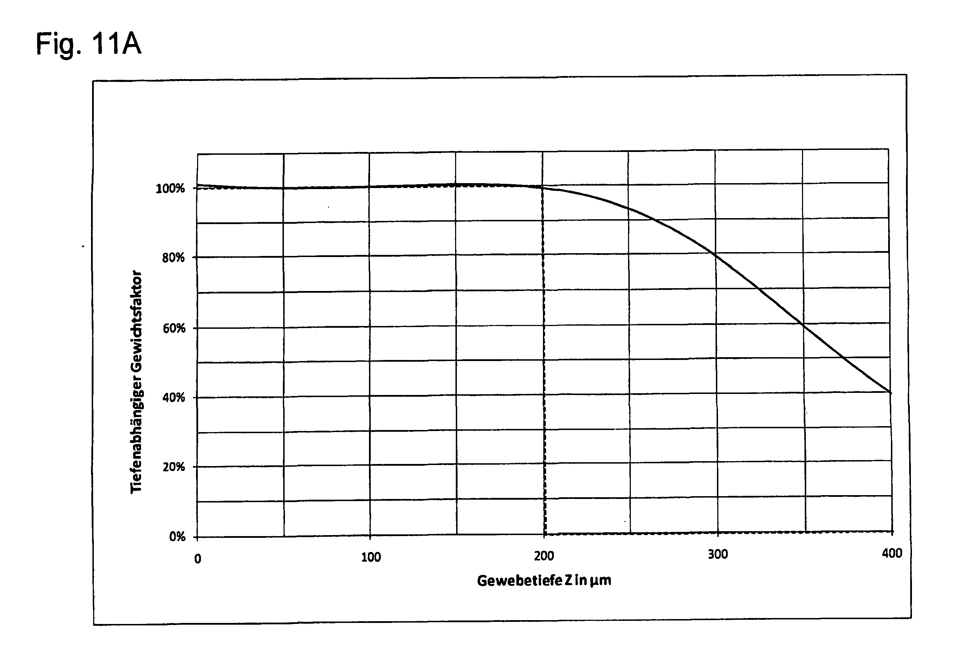

Fig. 11A zeigt qualitativ den tiefenabhängigen Gewichtsfaktor, der anzeigt, in welchem Maße bestimmte Tiefenbereiche zum gemessenen optischen Signal S beitragen. In diesem Fall ist die Fokustiefe Zfoc auf 300 µm eingestellt, die Integrationstiefe beträgt Zmax = 200µm. Für Tiefen kleiner als die hier gewählte Integrationstiefe (200 µm) ist der Beitrag im Wesentlichen konstant. Tieferliegende Bereiche tragen hingegen nur vermindert zum optischen Signal S bei.

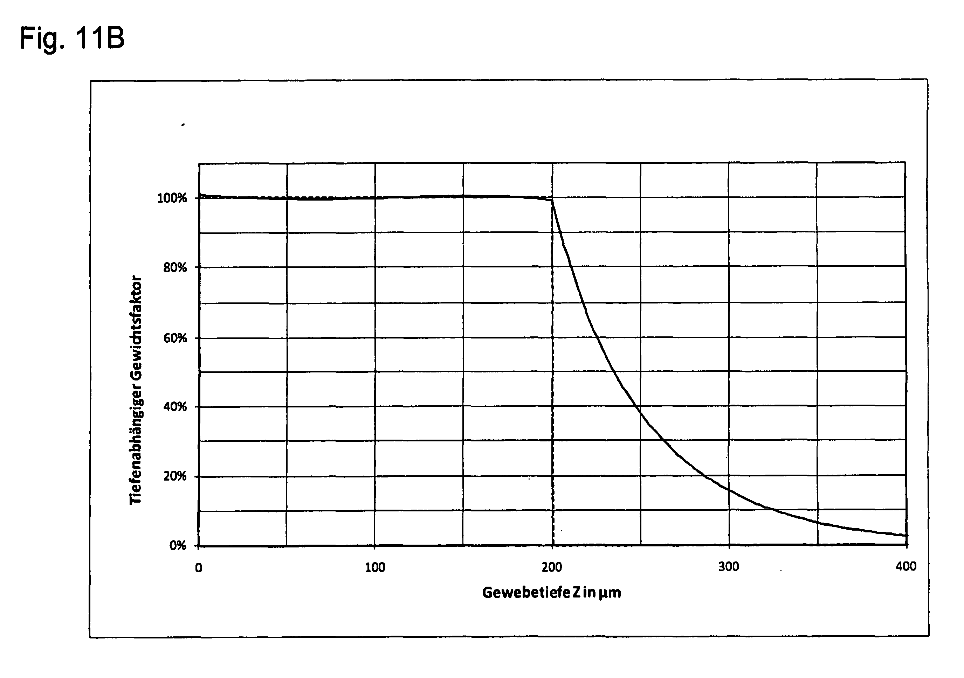

Die Integrationstiefe Zmax für das optische Signal S im Objekt 2 (Gewebe) wird weitgehend dadurch begrenzt, dass der Zwei-Photonen-Effekt nach Erreichen der Fokustiefe nicht mehr signalverstärkend wirkt (die maximale Integrationstiefe Zmax, bis zu der der Signalbeitrag tiefenunabhängig in etwa konstant ist, ist dabei kleiner als die Fokustiefe Zfoc). Durch optische Maßnahmen wie das Vorsehen einer Blende in der Kollektionsoptik (Kollektionseffizienzbegrenzung) kann dieser Abschneideeffekt noch verstärkt werden. Eine qualitative Darstellung des tiefenabhängigen Gewichtsfaktors bei Einsatz einer im Strahlengang des optischen Signals S angeordneten Blende zum Abschneiden von optischen Signalen S aus größeren Tiefen zeigt Fig. 11B.

Der Untersuchungsvorgang einer Läsion an einem Patienten unter Verwendung der Vorrichtung 1 gliedert sich grundlegend in zwei Abschnitte:
1. Aufnahme eines Realbildes der Oberfläche mittels der Realbildkamera 111 (Fig. 3 und 4) und Entscheidung über die detailliert zu untersuchenden Teilvolumina, incl. Festlegung der Aufnahmelinie (Scanspur) im Bild der Hautoberfläche;
2. Durchführung des Messvorgangs für die eigentliche Mikroskopie-Schnittaufnahme längs der im Realbild festgelegten Scanspur(en).

Das Realbild dient dabei als initiale Übersichtsaufnahme, um einem Nutzer die Auswahl einer geeigneten Aufnahmelinie zu ermöglichen, und liefert gleichzeitig zusätzliche dokumentierende klinische Information im Sinne der üblichen Dermoskopie.

Eine Untersuchung eines Patienten unter Verwendung der Vorrichtung 1 kann beispielsweise folgendermaßen ablaufen.

Eine Untersuchung beginnt mit der genauen Positionierung des am Tragarm 11 befestigten Patientenmoduls 10 (Messkopfes) am Patienten. Dabei wird die Realbildkamera 111 zur Suche verwendet, die bewegte Bilder der Hautoberfläche liefert. Dieses Video-Suchbild wird in einer Benutzeroberfläche auf dem Monitor 13 (Fig. 1) laufend angezeigt. Nach Arretierung des Patientenmoduls 10 wird eine einzelne Übersichtsaufnahme gemacht und im Rahmen eines Läsions-Datensatzes gespeichert.

Um einem Nutzer die Festlegung der Ebene für das aufzunehmende Schnittbild zu erleichtern, kann in der Übersichtsaufnahme der Benutzeroberfläche überlagert ein Auswahlgebiet eingeblendet werden, welches dem tatsächlichen Gebiet der folgenden Mikroskopie-Aufnahme entspricht und vom Nutzer in lateraler Länge und Lage definierbar und anpassbar ist. Zusätzlich muss der Nutzer die axiale Tiefe angeben, um ein zweidimensionales Gebiet für das aufzunehmende Schnittbild festzulegen.

Nach Definition der Lage des Schnittbildes kann zunächst eine Kalibrierungsaufnahme zur automatischen Einstellung der Laserleistung als Funktion der axialen Tiefe vorgenommen werden; erst anschließend erfolgt dann die eigentliche mikroskopische Aufnahme. Während der Aufnahme des Schnittbildes wird dabei das bereits erhaltene Bild laufend aktualisiert, um Fehlaufnahmen rechtzeitig abbrechen zu können. Nach Aufnahme des Schnittbildes kann der Nutzer weitere Bilder aufnehmen und zum Läsions-Datensatz hinzufügen.

Folgende Daten können beispielsweise zur Untersuchung einer Läsion eines Patienten erhoben werden:
- Realbild: 4 Megapixel Auflösung im qualitativ hochwertigem Fotomodus; minimal 1 Megapixel Auflösung im Videomodus, Bildfrequenz 25 fps; Belichtungszeit (im Fotomodus) 50 ms oder kürzer.
- Mikroskop-Bild: Der Nutzer definiert eine Strecke von 1-10 mm lateral mit einer maximalen Tiefe von 20 bis 150 µm axial, wobei eine Messung stets an der Hautoberfläche (bei einer Tiefe von 0 µm) beginnt. Für eine Läsion kann der Nutzer beliebig viele Mikroskopie-Aufnahmen durchführen.
- Um mögliche Verschiebungen gegenüber dem ersten Realbild erkennen zu können, wird ein zweites Realbild nach der eigentlichen Mikroskopie-Schnittaufnahme aufgenommen und mit dem ersten verglichen (so genanntes "Pre- Scan-Bild" und "Post-Scan-Bild").

Ein Messvorgang kann beispielsweise in logischer und zeitlicher Reihenfolge folgendermaßen ablaufen:
1. Initialisierung der Vorrichtung 1:
   - die Vorrichtung 1 wird eingeschaltet,
   - der Linearmotor, der die Optikeinheit 103 bewegt, wird in Startposition gebracht,
   - die Vorrichtung 1 wird für eine Aufnahme vorbereitet (Spannungen werden bereitgestellt, Komponenten geprüft und initialisiert, Betriebsbereitschaft wird hergestellt und signalisiert);
2. Start des Untersuchungsvorgangs:
   - Patientendaten werden ggf. vom Bediener eingegeben,
   - ein Messdatensatz wird ggf. vom Bediener angelegt;
3. Auswahl des Untersuchungsareals und Positionierung des Patientenmoduls 10:
   - der Bediener stellt geeignete Bedingung für eine Aufnahme her,
   - ein Videobild der Hautoberfläche wird angezeigt;
4. Aufnahme eines Realbild ("Pre-Scan-Bild"):
   - sobald die Positionierung feststeht (Tragarm 11 ist arretiert und Arretierung wird angezeigt), wird die Aufnahme ausgelöst und das Ergebnis dem Nutzer angezeigt,
   - der Bediener akzeptiert oder verwirft das Bild (im letzten Fall Wiederholung),
   - das akzeptierte Bild wird als "Pre-Scan-Bild" gespeichert, wobei die aufgenommenen Daten gleichzeitig für eine klinische Bewertung der Läsion durch den Arzt und zur Messfeldbestimmung dienen;
5. Festlegung der Aufnahmelinie (Scanspur) im Realbild der Hauptoberfläche:
   - im Realbild wird die Aufnahmelinie (= Schnittlinie des X-Z-Scanfeldes mit der Hautoberfläche) als Linie eingeblendet dargestellt,
   - die Aufnahmelinie kann vom Nutzer in Orientierung und Länge verändert werden, um die laterale Scan-Länge (Ausdehnung in X-Richtung) und Scan-Position zu bestimmen,
   - die axiale Scan-Tiefe (Ausdehnung in Z-Richtung) wird über ein Bedienelement (z.B. einen Schieberegler einer graphischen Nutzerschnittstelle) justiert, wobei beispielsweise Werte zwischen 0 und150 µm einstellbar sind;
6. Kalibrierung der Laserleistung:
   - nach Festlegung der Scan-Daten kann der Nutzer die Anpassung der Laserleistung in Abhängigkeit von der Scan-Tiefe automatisch bestimmen lassen oder auch manuell selbst definieren;
7. Durchführung der Scan-Routine:
   - durch Drücken eines "Start"-Knopfes wird der mikroskopische Scan gestartet, d.h.:
   - zunächst wird die Realbildkamera 111 (manuell oder automatisch) in eine Ausweichposition gebracht,
   - am Startpunkt der Aufnahmelinie beginnt die Optikeinheit 103 mit der Rasterung des Gewebes,
   - während des gesamten Scanablaufs werden Sicherheitsfunktionen (Unterbrechung, Not-Aus, Leistungsüberwachung) laufend überwacht und die Laserleistung gemäß vordefinierter Funktion (Kalibrierung) laufend angepasst,
   - simultan baut sich ein Grauwertbild des Mess-Signals in einem oder in mehreren Fenstern (die den unterschiedlichen Spektralkanälen zugeordnet sind) auf dem Monitor 13 auf,
   - die Scanroutine endet im Regelfall automatisch mit kompletter Rasterung des Scanfeldes und Fertigmeldung auf dem Monitor 13 (weiter in Punkt 8) oder
   - im Fehlerfall mit Abbruch und Fehlermeldung (weiter in Punkt 10);
8. Abschluss der Scanroutine im Regelfall:
   - nach Ende des Scanvorgangs wird in den Realbildmodus geschaltet, die Realbildkamera 111 in eine Aufnahmeposition gebracht und ein weiteres Realbild aufgenommen; dieses "Post-Scan-Bild" wird mit dem zugehörigen gespeicherten "Pre-Scan-Bild" verglichen, wobei nur bei ausreichend genauer Deckungsgleichheit das eigentliche Scan-Bild "unverwackelt" und verwendbar ist;
   - bei positivem Ausgang der Prüfung wird dem Nutzer angeboten, die Daten zu speichern bzw. mit Kommentar zu versehen (weiter mit Punkt 9) oder eine weitere Aufnahme an anderer oder selber Stelle durchzuführen (Rücksprung zu Punkt 3);
9. Abschluss des Untersuchungsvorgangs:
   - sollen keine weiteren Scans durchgeführt oder Daten bearbeitet werden, wird die Vorrichtung 1 in einen Ruhezustand versetzt (in dem die Motoren in Startposition, der Shutter geschlossen und die PMTs spannungslos sind) und das Anwendungsprogramm wird beendet.

Die Bedienungssteuerung kann durch eine Software realisiert sein, die in einem Anwendungsprogramm mit einer graphischen Nutzerschnittstelle (GUI) implementiert sein kann und folgende logische Funktionen aufweist:
- Initialisierung des Gesamtsystems, insbesondere Selbsttest der HW Komponenten und Anzeige von System-Status und Fehlern; Überwachungsfunktionen (Laserleistung; Laserzustand);
- Funktionen für die Verwaltung von Patientendaten, insbesondere eine entsprechende Ordner- und Datenbankstruktur, die die strukturierte Eingabe von patientenbezogenen Daten sowie zugehörige administrative Funktionen wie Suchen, Ändern, Ausdrucken und Löschen von Datensätzen ermöglicht;
- Funktionen für die Verwaltung von Messdaten (Realbild; Mikroskopbild) und deren Zuordnung zu Patientendatensätzen;
- Funktionen für die graphische Darstellung der Messdaten aus den unterschiedlichen spektralen Kanälen und Funktionen für die Weiterverarbeitung der Messdaten (z.B. Falschfarbendarstellung, Bereichsauswahl, Verhältnisbildung, Kantenerkennung etc.);
- Funktionen für die Steuerung des optischen und des mechanischen Systems;
- Funktionen für die allgemeine Ablaufsteuerung, z.B. Assistenten für die Nutzerführung bei Scans.

Die Steuer- und Verarbeitungseinheit 12 (siehe Fig. 1) weist zur Steuerung und Signalverarbeitung bevorzugt eine elektronische Schaltung auf, die einerseits Kontrolle über alle daran angeschlossenen Subsysteme hat und andererseits die Kommunikation mit der auf einem Rechner der Steuer- und Verarbeitungseinheit 12 installierten Anwendersoftware steuert. Durch die Zweiteilung einerseits der Steuerung der angeschlossenen Komponenten über die elektronische Schaltung und andererseits der Installation der Anwendungssoftware auf einem separaten Rechner (PC) besteht die Möglichkeit, einfache Signalverarbeitungsaufgaben und die Steuerung der angeschlossenen Komponenten wie Photodioden, Shutter und Schrittmotoren bzw. deren Treibern auf Seiten der elektronischen Schaltung in Echtzeit durchzuführen, während komplexere Aufgaben ohne Echtzeitanforderung und Sicherheitsrelevanz von dem Anwendungsprogramm auf dem Rechner durchgeführt werden. Für eine ausreichende Flexibilität sollte die Steuer- und Verarbeitungseinheit 12 programmierbar ausgelegt werden.

Zum Schutze der Detektoren 1221, 1222, 1223 (beispielsweise ausgeführt als PMTs) des Detektormoduls 121 kann vorgesehen sein, die Helligkeit außerhalb des Patientenmoduls 10 mittels einer Photodiode zu messen. Liegt diese über einem zulässigen Wert, d.h. wird potentiell zu viel Licht über die optische Faser 110 transportiert, wird eine Steuerspannung für die Detektoren 1221, 1222, 1223 herunter geregelt und eine Fehlermeldung an das Anwendungsprogramm gesendet.

Um eine Exposition des menschlichen Auges mit Laserlicht auszuschließen, kann vorgesehen sein, den zur Steuerung der Belichtung vorgesehenen Shutter erst dann zu öffnen, wenn eine mechanische (Schalter) oder optische (Lichtschranke) Vorrichtung einen engen Kontakt des Patientenmoduls 10 mit dem zu untersuchenden Objekt 2 signalisiert. Ob ein Kontakt vorliegt oder nicht, kann an das Anwendungsprogramm übermittelt werden.

Die Vorrichtung 1 als bildgebendes Medizinsystem stellt die gewonnenen Daten bildlich dar, wobei die Daten so zeitnah wie möglich (idealerweise in Echtzeit) verfügbar gemacht bzw. dargestellt werden. Systemzustände wie Fehlermeldungen werden dabei in entsprechenden Anzeigen erkennbar gemacht.

Eingabeseitig kann eine Anwendungssoftware dabei dazu ausgebildet sein, Nutzereingaben intuitiv und auf das jeweilige Objekt bezogen zu verarbeiten. Dies bedeutet insbesondere, dass die Positionierung eines Messfeldes (Region of Interest, ROI) direkt im vorab aufgenommenen Realbild erfolgen kann und dass eine Bereichsauswahl in einem Bild (Realbild oder Mikroskopbild) mit geeigneten Zoom- und Scrollfunktionen sowie Drag- und Drop unterstützt wird. Zudem können Werkzeuge vorgesehen sein, die einem Nutzer notwendige Berechnungen abnehmen und die Auswertung der Bilder (Messung von Größen und Abständen) unterstützen. Eine Untersuchung kann jederzeit über die Anwendungssoftware durch einen Nutzer unterbrochen oder abgebrochen werden. Je nach Betriebsart (Scan oder Auswertung) stellt die Software entsprechende Bedienmasken zur Verfügung.

Ausgabeseitig ist die Anwendungssoftware ausgebildet, jede Systemänderung zu melden und eine Bildaktualisierung unmittelbar umzusetzen. Hierzu gehört z.B. der Betriebszustand im Allgemeinen, der Bewegungszustand der Motoren, der Fortschritt eines Messprogramms und der zeitnahe Aufbau von Real- und Mikroskopbildern. Bedingt durch die Geometrie der aufgenommenen Schnittbilder (bei denen die Bildbreite ein Vielfaches der Bildhöhe ist, z.B. mit einem Verhältnis von ca. 1:50) ist es erforderlich, dass ein Mikroskopbild entweder in nebeneinander liegenden Teil-Streifen oder als (möglichst langer) Ausschnitt dargestellt wird.

Die Erfindung ist nicht auf die vorangehend geschilderten Ausführungsbeispiele beschränkt. Insbesondere sind die geschilderten Verfahren und Vorrichtungen grundsätzlich nicht auf die Zwei-Photonen-Anregung beschränkt, sondern können auch zur Drei- oder Mehr-Photonen-Mikroskopie oder -Spektroskopie eingesetzt werden.

### Bezugszeichenliste

- 1: Vorrichtung
- 10, 10': Patientenmodul
- 100: Vorrichtung zur Strahlkonditionierung
- 1001: Frequenzverdoppler
- 1002: Wellenlängenfilter
- 1003: Leistungseinsteller und -messer
- 1004: Teleskop zur Strahlanpassung
- 101, 102: Spiegel
- 103: Optikeinheit
- 104: Dichroitisches Element
- 105: Objektiv
- 106: Linse
- 107: Scheibe
- 108: Sperrfilter
- 109: Linse
- 110: Optische Faser
- 100': Linse
- 101': Frequenzverdoppler
- 102': Polarisator
- 103': Lambda-Viertel-Plättchen
- 104': Linse
- 105': Adaptiver Spiegel
- 106': Linse
- 107': Zwei-Achsen-Kipp-Spiegel
- 108': Teleskop
- 109': Linse
- 110': Strahlteiler
- 111': Linse
- 112': Detektor
- 113': Treiberelektronik
- 114': Detektorelektronik
- 115': Treiberelektronik
- 116': Optikeinheit
- 117': Linse
- 11: Tragarm
- 12: Steuer- und Verarbeitungseinheit
- 120: Lasereinheit
- 121: Detektormodul
- 1210: Linse
- 1211: Sperrfilter
- 1212, 1213: dichroitische Spiegel
- 1214, 1215, 1216: Sperrfilter
- 1218, 1219, 1220: Linse
- 1221, 1222, 1223: Detektor
- 122: Optische Faser
- 13: Monitor
- 2: Objekt
- 3: Bildebene
- 31: Aufnahmelinie
- A: Anregungsstrahl
- BX, BZ: Bewegung
- C: Umrechnung
- D1, D2, D3: Detektion
- Dfoc: Fokusdurchmesser
- E1, E2: Ergebnisbild
- FMV: Fluoreszenzmessvolumen
- GPO: Gewebe- bzw. Proben-Oberfläche
- L: Spektrum einer Läsion
- N: Spektrum der Normalhaut
- O: Optische Achse
- R: Rotationsachse
- S, S1, S2, S3: Signal
- Σ: Summenbildung
- V, V1, V2, V3: Verstärkung
- Zfoc: Fokustiefe
- Zmax: Tiefengrenze des Fluoreszenzmessvolumens

## Patentansprüche

1. Vorrichtung für die Mehr-Photonen-Fluoreszenzmikroskopie zur Gewinnung von Informationen aus biologischem Gewebe, mit
- einer Lasereinheit (120) zur Erzeugung einer Anregungsstrahlung (A),
- einer Optikeinheit (103), die ausgebildet ist, die Anregungsstrahlung (A) zur Erzeugung eines optischen Signals (S) an unterschiedlichen Orten in oder an einem zu untersuchenden Objekt (2) zu fokussieren, und
- einem Detektormodul (121) zur Erfassung des optischen Signals (S) aus dem Bereich des Objekts (2),
wobei die Optikeinheit (103) zur Erzeugung des optischen Signals (S) an unterschiedlichen Orten in oder an dem Objekt (2) zumindest in einer Richtung (X, Y, Z) relativ zum Objekt (2) beweglich ist,
**dadurch gekennzeichnet, dass**
- die Vorrichtung (1) eine Steuer- und Verarbeitungseinheit (12) und ein mit der Steuer- und Verarbeitungseinheit (12) verbundenes Patientenmodul (10), das zur Untersuchung des Objekts (2) relativ zum Objekt (2) platzierbar ist, aufweist, wobei die Optikeinheit (103) Teil des Patientenmoduls (10) ist,
- die Lasereinheit (120) eine Anregungsstrahlung (A) mit einer ersten Wellenlänge erzeugt und der Optikeinheit (103) ein Frequenzverdoppler (1001) zur Halbierung der Wellenlänge der Anregungsstrahlung (A) im Patientenmodul vorgeschaltet ist,
- das Patientenmodul (10) einen für die Anregungsstrahlung (A) und das optische Signal (S) durchlässigen Kontaktabschnitt (107) aufweist, der zur Untersuchung des Objekts (2) mit dem Objekt (2) in Kontakt zu bringen ist, wobei Mittel vorhanden sind, die die Optikeinheit (103) in horizontaler Richtung (X, Y) relativ zu dem Kontaktabschnitt (107) innerhalb des Patentientanmoduls (10) zur Generierung von Schnittbildern mit gegebener Kantenlänge bewegen,
- die Lasereinheit (120) Teil der Steuer- und Verarbeitungseinheit (12) ist, wobei eine optische Faser (122) die Lasereinheit (120) mit dem Patientenmodul (10) zur Übertragung der Anregungsstrahlung (A) hin zur Optikeinheit (103) verbindet, und
- die Optikeinheit (103) unabhängig von der optischen Faser (122) zur Übertragung der Anregungsstrahlung (A) beweglich ist.

2. Vorrichtung nach Anspruch 1, **dadurch gekennzeichnet, dass** bei einer Bewegung der Optikeinheit (103) zur Erzeugung des optischen Signals (S) die Winkellage der optischen Achse (O) der auf das Objekt (2) fallenden Anregungsstrahlung (A) sich nicht ändert.

3. Vorrichtung nach Anspruch 1 oder 2, **dadurch gekennzeichnet, dass** die Optikeinheit (103) in horizontaler Richtung (X, Y) und/oder in vertikaler Richtung (Z) relativ zu einer der Optikeinheit (103) zugewandten Oberfläche des Objekts (2) bewegbar ist.

4. Vorrichtung nach Anspruch 3, **dadurch gekennzeichnet, dass** die Optikeinheit (103) ein Objektiv (105) zur Fokussierung der Anregungsstrahlung (A) an einem Ort in oder an dem Objekt (2) aufweist, wobei das Objektiv (105) in vertikaler Richtung (Z) relativ zur Oberfläche des Objekts (2) bewegbar ist.

5. Vorrichtung nach einem der vorangehenden Ansprüche, **dadurch gekennzeichnet, dass** die Optikeinheit (103) zur pixelweisen Erzeugung eines vertikalen Schnittbildes zumindest abschnittsweise kontinuierlich in horizontaler Richtung (X, Y) und/oder in vertikaler Richtung (Z) relativ zu dem Objekt (2) verfahrbar ist.

6. Vorrichtung nach einem der vorangehenden Ansprüche, **dadurch gekennzeichnet, dass** die Lasereinheit (120) einen Ultrakurzpulslaser zur Erzeugung von Laserpulsen im Femtosekunden-Bereich aufweist.

7. Vorrichtung nach einem der vorangehenden Ansprüche, **dadurch gekennzeichnet, dass** die Optikeinheit (103) zur Kollektion des optischen Signals (S) ausgebildet ist und über eine optische Faser (110) mit dem Detektormodul (121) zur Übertragung des aufgenommenen optischen Signals (S) an das Detektormodul (121) verbunden ist.

8. Vorrichtung nach Anspruch 7, **dadurch gekennzeichnet, dass** die optische Faser (110) zur Übertragung des aufgenommenen optischen Signals (S) an das Detektormodul (121) sich von einer zur Übertragung der Anregungsstrahlung (A) vorgesehenen optischen Faser (122) unterscheidet.

9. Verfahren für die Mehr-Photonen-Fluoreszenzmikroskopie zur Gewinnung von Informationen aus biologischem Gewebe, bei dem
- eine Lasereinheit (120) eine Anregungsstrahlung (A) erzeugt,
- eine Optikeinheit (103) die Anregungsstrahlung (A) zur Erzeugung eines optischen Signals (S) an unterschiedlichen Orten in oder an einem zu untersuchenden Objekt (2) fokussiert und
- ein Detektormodul (121) das optische Signal (S) aus dem Bereich des Objekts (2) erfasst,
wobei die Optikeinheit (103) zur Erzeugung des optischen Signals (S) in oder an dem Objekt zumindest in einer Richtung (X, Y, Z) relativ zum Objekt (2) bewegt wird,
**dadurch gekennzeichnet, dass**
- die Vorrichtung (1) eine Steuer- und Verarbeitungseinheit (12) und ein mit der Steuer- und Verarbeitungseinheit (12) verbundenes Patientenmodul (10), das zur Untersuchung des Objekts (2) relativ zum Objekt (2) platzierbar ist, aufweist, wobei die Optikeinheit (103) Teil des Patientenmoduls (10) ist,
- die Lasereinheit (120) eine Anregungsstrahlung (A) mit einer ersten Wellenlänge erzeugt und der Optikeinheit (103) ein Frequenzverdoppler (1001) zur Halbierung der Wellenlänge der Anregungsstrahlung (A) im Patientenmodul vorgeschaltet ist,
- das Patientenmodul (10) einen für die Anregungsstrahlung (A) und das optische Signal (S) durchlässigen Kontaktabschnitt (107) aufweist, der zur Untersuchung des Objekts (2) mit dem Objekt (2) in Kontakt gebracht wird, wobei die Optikeinheit (103) in horizontaler Richtung (X, Y) relativ zu dem Kontaktabschnitt (107) innerhalb des Patentientanmoduls (10) zur Generierung von Schnittbildern mit gegebener Kantenlänge bewegt wird,
- die Lasereinheit (120) Teil der Steuer- und Verarbeitungseinheit (12) ist, wobei eine optische Faser (122) die Lasereinheit (120) mit dem Patientenmodul (10) zur Übertragung der Anregungsstrahlung (A) hin zur Optikeinheit (103) verbindet, und
- die Optikeinheit (103) unabhängig von der optischen Faser (122) zur Übertragung der Anregungsstrahlung (A) beweglich ist.

10. Verfahren nach Anspruch 9, **dadurch gekennzeichnet, dass** aus den empfangenen optischen Signalen (S) an unterschiedlichen Orten des Objekts (2) pixelweise ein Schnittbild des Objekts (2) erzeugt wird.

11. Verfahren nach Anspruch 9 oder 10, **dadurch gekennzeichnet, dass** zur pixelweisen Erzeugung des Schnittbilds das Objekt (2) in getriggerter Weise mit der Anregungsstrahlung (A) belichtet wird, wobei die Pixelgröße
- in horizontaler Richtung (X, Y) durch den Fokus der Anregungsstrahlung (A) und durch die Einstellung der Triggerung und
- in vertikaler Richtung (Z) durch die Taillenlänge der fokussierten Anregungsstrahlung (A)
bestimmt ist, wobei die Pixelgröße durch eine Strahlaufweitung der Anregungsstrahlung (A) und Einstellung der Triggerung einstellbar ist.

## Claims

1. A device for multi-photon fluorescence microscopy for obtaining information from biological tissue, comprising
- a laser unit (120) for generating an excitation radiation (A),
- an optical unit (103) which is formed to focus the excitation radiation (A) for generating an optical signal (S) at different locations in or on an object (2) to be examined, and
- a detector module (121) for detecting the optical signal (S) from the region of the object (2),
wherein the optical unit (103) for generating the optical signal (S) at different locations in or on the object (2) is movable in at least one direction (X, Y, Z) relative to the object (2)
**characterized in that**
- the device (1) includes a control and processing unit (12) and a patient module (10) connected with the control and processing unit (12), which for examining the object (2) can be placed relative to the object (2), wherein the optical unit (103) is part of the patient module (10),
- the laser unit (120) generates an excitation radiation (A) with a first wavelength and in the patient module (10) a frequency doubler (1001) for halving the wavelength of the excitation radiation (A) is connected before of the optical unit (103),
- the patient module (10) includes a contact portion (107) transmissive for the excitation radiation (A) and the optical signal (S), which for examining the object (2) must be brought in contact with the object (2), wherein means are provided which move the optical unit (103) in horizontal direction (X, Y) relative to the contact portion (107) within the patient module (10) for generation of sectional images with given edge length,
- the laser unit (120) is part of the control and processing unit (12), wherein an optical fiber (122) connects the laser unit (120) with the patient module (10) for transmitting the excitation radiation (A) towards the optical unit (103), and
- the optical unit (103) is movable independent of the optical fiber (122) for transmitting the excitation radiation (A).

2. The device according to claim 1, **characterized in that** during a movement of the optical unit (103) for generating the optical signal (S) the angular position of the optical axis (O) of the excitation radiation (A) falling onto the object (2) is not changed.

3. The device according to claim 1 or 2, **characterized in that** the optical unit (103) is movable in horizontal direction (X, Y) and/or in vertical direction (Z) relative to a surface of the object (2) facing the optical unit (103).

4. The device according to claim 3, **characterized in that** the optical unit (103) includes an objective (105) for focusing the excitation radiation (A) at a location in or on the object (2), wherein the objective (105) is movable in vertical direction (Z) relative to the surface of the object (2).

5. The device according to any of the preceding claims, **characterized in that** for generating a vertical sectional image pixel by pixel the optical unit (103) is at least partly continuously movable in horizontal direction (X, Y) and/or in vertical direction (Z) relative to the object (2).

6. The device according to any of the preceding claims, **characterized in that** the laser unit (120) includes an ultra-short pulse laser for generating laser pulses in the femtosecond range.

7. The device according to any of the preceding claims, **characterized in that** the optical unit (103) is formed for collecting the optical signal (S) and via an optical fiber (110) the optical unit (103) is connected with the detector module (121) for

8. The device according to claim 7, **characterized in that** the optical fiber (110) for transmitting the recorded optical signal (S) to the detector module (121) differs from an optical fiber (122) provided for transmitting the excitation radiation (A).

9. A method for multi-photon fluorescence microscopy for obtaining information from biological tissue, in which
- a laser unit (120) generates an excitation radiation (A),
- an optical unit (103) focuses the excitation radiation (A) for generating an optical signal (S) at different locations in or on an object (2) to be examined, and
- a detector module (121) detects the optical signal (S) from the region of the object (2),
wherein the optical unit (103) for generating the optical signal (S) in or on the object is moved in at least one direction (X, Y, Z) relative to the object (2),
**characterized in that**
- the device (1) includes a control and processing unit (12) and a patient module (10) connected with the control and processing unit (12), which for examining the object (2) can be placed relative to the object (2), wherein the optical unit (103) is part of the patient module (10),
- the laser unit (120) generates an excitation radiation (A) with a first wavelength and in the patient module (10) a frequency doubler (1001) for halving the wavelength of the excitation radiation (A) is connected before of the optical unit (103),
- the patient module (10) includes a contact portion (107) transmissive for the excitation radiation (A) and the optical signal (S), which for examining the object (2) is brought in contact with the object (2), wherein the optical unit (103) is moved in horizontal direction (X, Y) relative to the contact portion (107) within the patient module (10) for generation of sectional images with given edge length,
- the laser unit (120) is part of the control and processing unit (12), wherein an optical fiber (122) connects the laser unit (120) with the patient module (10) for transmitting the excitation radiation (A) towards the optical unit (103), and
- the optical unit (103) is movable independent of the optical fiber (122) for transmitting the excitation radiation (A).

10. The method according to claim 9, **characterized in that** from the received optical signals (S) a sectional image of the object (2) is generated pixel by pixel at different locations of the object (2).

11. The method according to claim 9 or 10, **characterized in that** for generating the sectional image pixel by pixel the object (2) is exposed to the excitation radiation (A) in a triggered manner, wherein the pixel size is determined
- in horizontal direction (X, Y) by the focus of the excitation radiation (A) and by adjusting the triggering, and
- in vertical direction (Z) by the waist length of the focused excitation radiation (A),
wherein the pixel size is adjustable by a beam expansion of the excitation radiation (A) and by adjusting the triggering.

## Revendications

1. Dispositif pour la microscopie par fluorescence multiphotonique visant à recueillir des informations de tissus biologiques, comprenant
- une unité laser (120) servant à produire un rayonnement d'excitation (A),
- une unité optique (103) qui est réalisée afin de focaliser le rayonnement d'excitation (A) servant à produire un signal optique (S) sur divers emplacements dans ou au niveau d'un objet (2) à examiner, et
- un module détecteur (121) servant à détecter le signal optique (S) en provenance de la zone de l'objet (2),
l'unité optique (103) servant à produire le signal optique (S) en divers emplacements dans ou au niveau de l'objet (2) étant mobile au moins dans une direction (X, Y, Z) par rapport à l'objet (2),
**caractérisé en ce que**
- le dispositif (1) présente une unité de commande et de traitement (12) et un module pour patient (10) relié à l'unité de commande et de traitement (12), lequel module pour patient peut être placé par rapport à l'objet (2) afin d'examiner l'objet (2), l'unité optique (103) faisant partie du module pour patient (10),
l'unité laser (120) produit un rayonnement d'excitation (A) présentant une première longueur d'onde, et un doubleur de fréquence (1001) servant à diviser par deux la longueur d'onde du rayonnement d'excitation (A) dans le module pour patient est installé en amont de l'unité optique (103),
- le module pour patient (10) présente une section de contact (107) laissant passer le rayonnement d'excitation (A) et le signal optique (S), laquelle est amenée en contact avec l'objet (2) aux fins de l'examen de l'objet (2), des moyens étant présents déplaçant l'unité optique (103) dans la direction horizontale (X, Y) par rapport à la section de contact (107) à l'intérieur du module pour patient afin de générer des images de coupe présentant une cote donnée,
- l'unité laser (120) fait partie de l'unité de commande et de traitement (12), une fibre optique (122) reliant l'unité laser (120) au module pour patient (10) aux fins de la transmission du rayonnement d'excitation (A) en direction de l'unité optique (103), et
- l'unité optique (103) est mobile indépendamment de la fibre optique (122) servant à transmettre le rayonnement d'excitation (A).

2. Dispositif selon la revendication 1, **caractérisé en ce que** dans le cas d'un déplacement de l'unité optique (103) servant à produire le signal optique (S), la position angulaire de l'axe optique (O) du rayonnement d'excitation (A) arrivant sur l'objet (2) ne varie pas.

3. Dispositif selon la revendication 1 ou 2, **caractérisé en ce que** l'unité optique (103) peut être déplacée dans la direction horizontale (X, Y) et/ou dans la direction verticale (Z) par rapport à une surface, tournée vers l'unité optique (103), de l'objet (2).

4. Dispositif selon la revendication 3, **caractérisé en ce que** l'unité optique (103) présente un objectif (105) servant à focaliser le rayonnement d'excitation (A) sur un emplacement dans ou au niveau de l'objet (2), l'objectif (105) pouvant être déplacé dans la direction verticale (Z) par rapport à la surface de l'objet (2).

5. Dispositif selon l'une quelconque des revendications précédentes, **caractérisé en ce que** l'unité optique (103) servant à produire de manière pixélisée une image de coupe verticale peut être déplacée au moins par endroits en continu dans la direction horizontale (X, Y) et/ou dans la direction verticale (Z) par rapport à l'objet (2).

6. Dispositif selon l'une quelconque des revendications précédentes, **caractérisé en ce que** l'unité laser (120) présente un laser à impulsions ultra courtes servant à produire des impulsions laser relevant de la plage des femtosecondes.

7. Dispositif selon l'une quelconque des revendications précédentes, **caractérisé en ce que** l'unité optique (103) est réalisée afin de collecter le signal optique (S) et est reliée, par l'intermédiaire d'une fibre optique (110), au module détecteur (121) aux fins de la transmission au module détecteur (121) du signal optique (S) reçu.

8. Dispositif selon la revendication 7, **caractérisé en ce que** la fibre optique (110) servant à transmettre au module détecteur (121) le signal optique (S) reçu se distingue d'une fibre optique (122) prévue aux fins de la transmission du rayonnement d'excitation (A).

9. Procédé pour la microscopie par fluorescence multiphotonique visant à recueillir des informations de tissus biologiques, dans le cadre duquel
- une unité laser (120) produit un rayonnement d'excitation (A),
- une unité optique (103) focalise le rayonnement d'excitation (A) servant à produire un signal optique (S) sur divers emplacements dans ou au niveau de l'objet (2) à examiner, et
- un module détecteur (121) détecte le signal optique (S) en provenance de la zone de l'objet (2),
l'unité optique (103) servant à produire le signal optique (S) dans ou au niveau de l'objet étant déplacée au moins dans une direction (X, Y, Z) par rapport à l'objet (2), **caractérisé en ce que**
- le dispositif (1) présente une unité de commande et de traitement (12) et un module pour patient (10) relié à l'unité de commande et de traitement (12), lequel module pour patient peut être placé par rapport à l'objet (2) afin d'examiner l'objet (2), l'unité optique (103) faisant partie du module pour patient (10),
l'unité laser (120) produit un rayonnement d'excitation (A) présentant une première longueur d'onde, et un doubleur de fréquence (1001) servant à diviser par deux la longueur d'onde du rayonnement d'excitation (A) dans le module pour patient est installé en amont de l'unité optique (103),
- le module pour patient (10) présente une section de contact (107) laissant passer le rayonnement d'excitation (A) et le signal optique (S), laquelle est amenée en contact avec l'objet (2) aux fins de l'examen de l'objet (2), l'unité optique (103) étant déplacé dans la direction horizontale (X, Y) par rapport à la section de contact (107) à l'intérieur du module pour patient afin de générer des images de coupe présentant une cote donnée,
- l'unité laser (120) fait partie de l'unité de commande et de traitement (12), une fibre optique (122) reliant l'unité laser (120) au module pour patient (10) aux fins de la transmission du rayonnement d'excitation (A) en direction de l'unité optique (103), et
- l'unité optique (103) est mobile indépendamment de la fibre optique (122) servant à transmettre le rayonnement d'excitation (A).

10. Procédé selon la revendication 9, **caractérisé en ce qu'**une image de coupe de l'objet (2) est produite de manière pixélisée à partir de signaux optiques (S) reçus en divers emplacements de l'objet (2).

11. Procédé selon la revendication 9 ou 10, **caractérisé en ce que** l'objet (2) est exposé au rayonnement d'excitation (A) de manière déclenchée afin de produire de manière pixélisée l'image de coupe, sachant que la taille des pixels est déterminée
- dans la direction horizontale (X, Y) par la focalisation du rayonnement d'excitation (A) et par le réglage du déclenchement et
- dans la direction verticale (Z) par la longueur en termes de taille du rayonnement d'excitation (A) concentré,
la taille des pixels pouvant être réglée par un élargissement des rayons du rayonnement d'excitation (A) et par le réglage du déclenchement.
